# EUROPEAN PATENT APPLICATION

(11) **EP 3 603 498 A1**
(43) Date of publication of application: **05.02.2020**
(21) Application number: 18774973.4
(22) Date of filing: 27.03.2018
(51) Int. Cl.: A61B 5/03, A61B 5/00

(54) **MONITORING APPARATUS, MONITORING BOUGIE, AND MONITORING SYSTEM**

(30) Priority: 28.03.2017 CN 201720316711 U; 28.03.2017 CN 201720312309 U; 28.03.2017 CN 201720312301 U; 28.03.2017 CN 201720312299 U; 28.03.2017 CN 201720311827 U; 28.03.2017 CN 201710194077; 28.03.2017 CN 201710194076; 28.03.2017 CN 201710193705; 28.03.2017 CN 201710193569; 28.03.2017 CN 201710193122
(71) Applicant: Pionmedek Medical Technologies Co., Ltd., Guangzhou, Guangdong 510663 (CN)
(72) Inventor: WEN, Ping, Guangzhou Guangdong 510663 (CN); LONG, Xiaoyan, Guangzhou Guangdong 510663 (CN); LAI, Yanqing, Guangzhou Guangdong 510663 (CN)
(74) Representative: Horn Kleimann Waitzhofer Patentanwälte PartG mbB
(86) International application number: PCT/CN2018/080705
(87) International publication number: WO 2018/177292

(57) **Abstract**

A monitoring system includes a monitoring apparatus (2) and a monitoring bougie (1). The monitoring apparatus (2) includes a first interface (21) detachably connecting the monitoring bougie (1), a signal processing module which receives and processes a collected signal transmitted by the monitoring bougie (1) to obtain a processed signal, and a display module which displays the processed signal. Since the monitoring bougie (1) and the monitoring apparatus (2) are connected detachably, the monitoring apparatus (2) can be removed from the monitoring bougie (1) during CT, MR or other inspection that affects or is affected by an electronic circuit, thereby causing no influence on the inspection result.

## Description

### TECHNICAL FIELD

The present disclosure relates to the technical field of healthcare, in particular to a monitoring apparatus, a monitoring bougie, and a monitoring system.

### BACKGROUND

The monitoring of physiological parameters in a living body (animal body or human body) is of great significance in clinical medicine and zoology. Traditional physiological parameter measurement relies on advanced sensor component technology, for example, measuring physiological parameters such as intracranial pressure, intracranial temperature, intracranial oxygen partial pressure, intravascular pressure, etc., and transferring the measured physiological parameters to a signal processing device for further processing and display, and so on. Generally, a multi-parameter bougie can integrate the measurement of various physiological parameters and transfer data to a corresponding monitor.

The physiological parameter monitoring system currently used has many limitations. For example, the current monitoring bougie can only be used in cooperation with a monitor, and the use is thus limited; data in the monitoring apparatus and the monitor can easily be deleted by mistake; the circuit of the monitoring apparatus (for example, an A/D converter, a signal amplifier, wired or wireless, etc.) interferes with magnetic resonance (MR) and computed tomography (CT) monitoring, resulting in loss of monitoring data (some important information is not very well protected); and there are errors in the monitoring data.

### SUMMARY

In view of the above, the present disclosure proposes a monitoring apparatus comprising: a first interface capable of detachably connecting to a monitoring bougie; a signal processing module configured to receive and process a collected signal transmitted by the monitoring bougie to obtain a processed signal; and a display module configured to display the processed signal.

In a possible embodiment, the monitoring apparatus further comprising a control panel configured to enable an operator to set one or more of zero setting data, patient information, an operation mode, a warning threshold value, and warning cancellation/delay.

In a possible embodiment, the monitoring apparatus further comprises one or both of a wired communication module capable of communication with a first terminal in a wired manner and a first wireless communication module capable of communication with the first terminal in a wireless manner.

In a possible embodiment, the control panel comprises an operation mode selector switch configured to control a communication method between the monitoring apparatus and the first terminal.

In a possible embodiment, the monitoring apparatus further comprises a first storage module configured to store the processed signal when communication with the first terminal is interrupted.

In a possible embodiment, the monitoring apparatus further comprises a first warning module configured to generate a warning signal to issue a warning according to a relationship between the processed signal and a warning threshold value.

In a possible embodiment, the first warning module is configured to issue no warning when a communication connection is established between the monitoring apparatus, and the first terminal comprises a second warning module configured to, when the communication connection is established between the monitoring apparatus and the first terminal, generate a warning signal to issue a warning according to the relationship between the processed signal and the warning threshold value, or issue a warning according to a warning signal provided by the monitoring apparatus.

In a possible embodiment, the monitoring apparatus further comprises a power module configured to supply power for the monitoring apparatus and the monitoring bougie.

In a possible embodiment, the monitoring apparatus has a dimension of a length ≤ 7 cm, a width ≤ 5 cm, and a thickness ≤ 2 cm.

In a possible embodiment, the display module is further configured to display one or more of data on the collected signal, a waveform of the processed signal, error information, a power level, warning information, service time of the monitoring bougie, an ID of monitoring bougie, patient information, and information on the wired/wireless connection.

In a possible embodiment, the monitoring apparatus and the first terminal are configured to establish a communication connection in a broadcast or handshake manner.

In a possible embodiment, the monitoring apparatus further comprises a voice input module, the control panel further comprises a voice button, and the voice button and the voice input module are configured to record a voice.

In a possible embodiment, the monitoring apparatus is wearable and is configured to fix to a head or a body of a monitored object by means of an attachment accessory.

In a possible embodiment, the monitoring apparatus further comprises: a receiving module configured to receive the collected signal transmitted by the monitoring bougie; and a gating module connected to the receiving module, the wired communication module, and the first wireless communication module and configured to transfer the collected signal received by the receiving module to one of the wired communication module and the first wireless communication module, so as to transmit the collected signal to the first terminal.

In a possible embodiment, the gating module comprises: a first detection module configured to detect whether a wired communication connection between the wired communication module and the first terminal is normal; and a first selection module connected to the first detection module and configured to transfer the collected signal to the wired communication module if the first detection module detects that the wired communication connection between the wired communication module and the first terminal is normal.

In a possible embodiment, the gating module comprises: an enable/disable module connected to the first detection module and configured to enable the first wireless communication module and transmit a command to a second detection module if the first detection module detects that the wired communication connection between the wired communication module and the first terminal is abnormal; and the second detection module connected to the enable/disable module and configured to, in response to the command from the enable/disable module, detect whether the wireless communication connection between the first wireless communication module and the first terminal is normal.

In a possible embodiment, the gating module comprises a second selection module connected to the second detection module and configured to transfer the collected signal to the first wireless communication module if the second detection module detects that the wireless communication connection between the first wireless communication module and the first terminal is normal.

In a possible embodiment, the enable/disable module is connected to the second detection module and is configured to disable the first wireless communication module if the second detection module detects that the wireless communication connection between the first wireless communication module and the first terminal is abnormal.

In a possible embodiment, the monitoring apparatus further comprises an adjustment module connected to the first detection module, the second detection module, and the receiving module, the adjustment module configured to cause the receiving module to sample the collected signal at a first sampling frequency if the first detection module detects that the wired communication connection between the wired communication module and the first terminal is normal.

The adjustment module is configured to cause the receiving module to sample the collected signal at a second sampling frequency if the first detection module detects that the wired communication connection between the wired communication module and the terminal is abnormal and the second detection module detects that the wireless communication connection between the first wireless communication module and the terminal is normal, wherein the first sampling frequency is higher than the second sampling frequency.

In a possible embodiment, the enable/disable module is configured to disable the first wireless communication module and regularly enable the first wireless communication module if the second detection module detects that the wireless communication connection between the first wireless communication module and the first terminal is abnormal.

In a possible embodiment, the first wireless communication module includes a Bluetooth communication module.

According to an aspect of the present disclosure, there is provided a monitoring bougie, comprising: a monitoring probe configured to acquire a collected signal; a connector, one end of the connector being connected to the monitoring probe via a first wire, and one other end of the connector has a second interface detachably connectable to the first interface of the monitoring apparatus described above; a second storage module configured to store an ID of the monitoring bougie and a preset threshold value.

In a possible embodiment, the monitoring probe includes a sensor configured to collect the collected signal.

In a possible embodiment, the second storage module is disposed in the monitoring probe, or in the connector, or between the monitoring probe and the connector.

In a possible embodiment, the monitoring probe has a diameter ≤800 µm.

In a possible embodiment, the sensor is a piezoelectric or piezoresistive sensor, a circuit of the sensor is of a full-bridge connection or a half-bridge connection, and the sensor is configured to measure an absolute pressure or a relative pressure.

In a possible embodiment, the second storage module is a memory chip not affected by MR and CT.

In a possible embodiment, the second storage module is further configured to store one or more of zero setting data, patient information, time of embedding, a factory version of monitoring bougie, and the collected signal.

In a possible embodiment, the monitoring bougie has an electronic identity tag.

In a possible embodiment, the monitoring probe further comprises a housing, a window being opened on a surface of the housing; the sensor being provided at a position facing the window.

In a possible embodiment, a packaging layer is provided at the window.

In a possible embodiment, one or more windows are opened on the surface of the housing.

In a possible embodiment, one or more sensors are provided at a position facing the same window.

In a possible embodiment, the sensor is provided at each of positions facing a plurality of windows.

In a possible embodiment, the plurality of sensors are separate from one another.

In a possible embodiment, the plurality of sensors are integrated as one component.

In a possible embodiment, the one or more windows are disposed on one or more side surfaces of the housing.

In a possible embodiment, the windows are disposed on opposite side surfaces of the housing.

In a possible embodiment, the windows are disposed on opposite side surfaces of the housing in a staggered arrangement.

In a possible embodiment, the window is disposed on a leading end of the housing.

In a possible embodiment, a packaging layer provided at the window disposed on the leading end has a streamline profile.

In a possible embodiment, a hollow cavity in communication with the window is provided in the housing.

In a possible embodiment, the monitoring bougie further comprises a base, the base being provided in the hollow cavity, the sensor being provided on the base.

In a possible embodiment, a first window is opened in a side surface of the housing, the base being provided in the hollow cavity on a side facing the first window.

In a possible embodiment, the base is of an L-shaped structure, the L-shaped structure including a vertical portion and a horizontal portion connected with each other, the horizontal portion being disposed below the first window, the sensor being provided on the horizontal portion.

In a possible embodiment, a second window is opened on the leading end of the housing.

In a possible embodiment, the monitoring bougie further comprises a base, the base being provided at the second window, the sensor being provided on the base.

In a possible embodiment, the base is of a plate-like structure matching the second window.

In a possible embodiment, the base is provided with a through hole in communication with the hollow cavity.

In a possible embodiment, the through hole is penetrated by a wire/optical fiber connected with the sensor.

In a possible embodiment, the base partially covers the second window.

In a possible embodiment, the base is provided with no hole.

In a possible embodiment, the sensor is fitted at the second window.

In a possible embodiment, there is provided a second wireless communication module close to one end of the monitoring bougie and connected to the monitoring probe, configured to wirelessly transmit the collected signal.

In a possible embodiment, there is provided a processing module connected to the monitoring probe and the second wireless communication module and configured to process the collected signal acquired by the monitoring probe and provide the processed collected signal to the second wireless communication module.

According to a further aspect of the present disclosure, there is provided a monitoring system, comprising: a first terminal, the monitoring apparatus described above, and the monitoring bougie described above, wherein the monitoring apparatus is configured to receive and process a collected signal transmitted by the monitoring bougie to obtain a processed signal, and transmit the processed signal to the first terminal, and the first terminal is configured to receive the processed signal from the monitoring apparatus, analyze the processed signal to acquire monitoring data, and display the monitoring data.

In a possible embodiment, the monitoring system further comprises: a server, the server connecting to the first terminal and receiving the monitoring data transmitted by the first terminal.

In a possible embodiment, the server connects to the second terminal, and the second terminal is capable of acquiring the monitoring data through the server.

According to a further aspect of the present disclosure, there is provided a drainage device, comprising: a drainage tube, the monitoring bougie described above, a catheter, and a drainage bottle. The drainage tube includes a tube body, a first through hole and a second through hole being provided on a side face of the tube body. The tube body is provided therein two or more cavities, at least one of the cavities being a wire cavity and at least one of the cavities being a drainage cavity. The first through hole is communicated with the wire cavity, and the second through hole is communicated with the drainage cavity. The monitoring bougie is provided in the wire cavity of the drainage tube and connects to the monitoring apparatus described above by means of a second wire provided in the wire cavity. The catheter connects the drainage tube with the drainage bottle.

In a possible embodiment, a leading end portion of the tube body has a streamline profile.

In a possible embodiment, the wire cavity is or is not in communication with the drainage cavity.

In a possible embodiment, the drainage tube has an outer diameter of 2.8 mm to 5.0 mm; the wire cavity has an inner diameter of 0.4 mm to 1.5 mm; and the drainage cavity has an inner diameter of 1.0 mm to 3.0 mm.

In a possible embodiment, the drainage tube is provided therein a support means.

In a possible embodiment, the support means is provided in a cavity in no communication with the drainage cavity and the wire cavity.

In a possible embodiment, an end of the support means extends out of the drainage tube through a hole in a wall of the leading end portion of the tube body.

In a possible embodiment, the drainage tube is provided with a third through hole in communication with the wire cavity, the third through hole used for leading the second wire.

In a possible embodiment, the leading end portion of the tube body is provided with graduations.

By the detachable connection between the monitoring bougie and the monitoring apparatus, when CT, MR, or other inspection that affects or is affected by an electronic circuit, is performed for a living body, the monitoring apparatus may be removed from the monitoring bougie so as to perform the inspection, which will not affect the inspection result.

By providing in the monitoring bougie a second storage module, it is possible to backup plural kinds of data, realizing independence of the monitoring bougie and the monitoring apparatus. The monitoring bougie removed from monitoring apparatus is connectable with another monitoring apparatus. It is possible that the monitoring apparatus continues the monitoring by identifying information (ID of the monitoring bougie) recorded in the memory chip of the monitoring bougie.

By separately providing the wire cavity and the drainage cavity, the drainage device according to the present disclosure avoids blockage of the drainage cavity caused by the monitoring means or a wire connecting the monitoring means, which affects the drainage effect. Further, the monitoring means is made sure to sufficiently contact the environment in the tissue, so as to accurately monitor the parameters in the tissue and prevent the body fluid in the drainage cavity from affecting the monitoring of the parameters.

Additional features and aspects of the present disclosure will become apparent from the following description of exemplary examples with reference to the attached drawings.

### BRIEF DESCRIPTION OF THE DRAWINGS

The drawings, which are incorporated in and constitute part of the specification, together with the description, illustrate exemplary examples, features and aspects of the present disclosure and serve to explain the principles of the present disclosure.
Fig. 1 is a schematic diagram of the monitoring apparatus according to an embodiment of the present disclosure.
Fig. 2 is a schematic diagram of the connection between the monitoring bougie and the monitoring apparatus according to an embodiment of the present disclosure.
Fig. 3 is a block diagram of the monitoring apparatus according to an embodiment of the present disclosure.
Fig. 4 is a schematic diagram of the monitoring system according to an embodiment of the present disclosure.
Fig. 5 is a block diagram of the monitoring apparatus according to an embodiment of the present disclosure.
Fig. 6 is a schematic diagram of the monitoring apparatus according to an embodiment of the present disclosure.
Fig. 7 is a schematic diagram of the monitoring bougie according to an embodiment of the present disclosure.
Figs. 8a and 8b are structural charts of the monitoring probe according to an embodiment of the present disclosure.
Fig. 9 is a schematic diagram of the monitoring probe according to an embodiment of the present disclosure.
Fig. 10 is a schematic diagram of the monitoring probe according to an embodiment of the present disclosure.
Figs. 11a to 11c and Figs. 12a to 12b are schematic diagrams of configuration of the windows of the monitoring probe according to an embodiment of the present disclosure.
Fig. 13 is a schematic diagram of the monitoring probe according to an embodiment of the present disclosure.
Figs. 14a to 14c are schematic diagrams of configuration of the packaging layer according to an embodiment of the present disclosure.
Figs. 15a to 15d are structural charts of the monitoring probe according to an embodiment of the present disclosure.
Fig. 16 is a structural chart of the monitoring probe according to an embodiment of the present disclosure.
Figs. 17 to 20 are structural charts of the monitoring probe according to an embodiment of the present disclosure.
Figs. 21a to 21c are structural charts of the base on the leading end of the monitoring probe according to an embodiment of the present disclosure.
Fig. 22 is a schematic diagram of the drainage tube according to an embodiment of the present disclosure.
Fig. 23 is a schematic diagram of the drainage tube used for drainage according to an embodiment of the present disclosure.
Figs. 24a and 24b are sectional views of the drainage tube according to an embodiment of the present disclosure.
Fig. 25 is a schematic diagram of the drainage tube according to an embodiment of the present disclosure.
Fig. 26 is a schematic diagram of the drainage device according to an embodiment of the present disclosure.
Fig. 27 is a schematic diagram of the drainage device according to an embodiment of the present disclosure.

1 monitoring bougie; 11 monitoring probe; 111 housing; 1111 window; 1112 packaging layer; 112 sensor; 12 connector; 122 second interface; 13 first wire;
2 monitoring apparatus; 21 first interface; 20 receiving module; 22 wired communication module; 23 first wireless communication module; 24 gating module; 241 first detection module; 242 first selection module; 243 enable/disable module; 244 second detection module; 245 second selection module; 26 adjustment module;
3 first terminal; 4 hollow cavity; 51 third window; 52 fourth window; 53 first window; 14 second window;
6 base; 61 vertical portion; 62 horizontal portion;
7 drainage tube; 71 tube body; 72 first through hole; 73 second through hole; 74 wire cavity; 741 wire; 75 drainage cavity; 76 third through hole;
8 catheter; 9 drainage bottle; 91 support means; 92, cavity; 93 Y-shaped member; 94 first triple valve; 95 second triple valve; 96 drainage bag; 97 supporting holder.

### DETAILED DESCRIPTION

Various exemplary examples, features and aspects of the present disclosure will be described in detail with reference to the drawings. The same reference numerals in the drawings represent parts having the same or similar functions. Although various aspects of the examples are shown in the drawings, it is unnecessary to proportionally draw the drawings unless otherwise specified.

Herein the term "exemplary" means "used as an instance or example, or explanatory". An "exemplary" example given here is not necessarily construed as being superior to or better than other examples.

Numerous details are given in the following examples for the purpose of better explaining the present disclosure. It should be understood by a person skilled in the art that the present disclosure can still be realized even without some of those details. In some of the examples, methods, means, elements and circuits that are well known to a person skilled in the art are not described in detail so that the principle of the present disclosure become apparent.

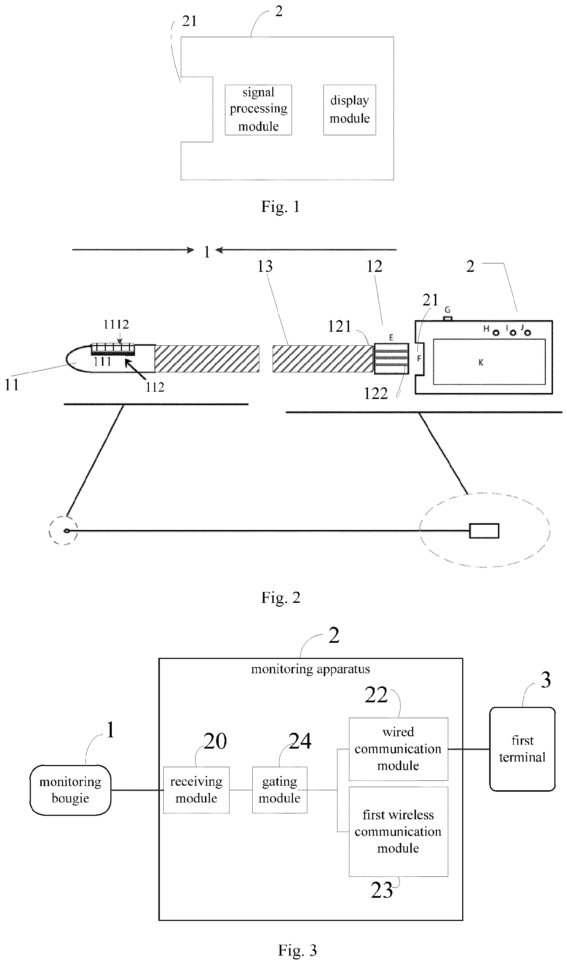

Fig. 1 is a schematic diagram of the monitoring apparatus according to an embodiment of the present disclosure. Fig. 2 is a schematic diagram of the connection between the monitoring bougie 1 and the monitoring apparatus 2 according to an embodiment of the present disclosure. As shown in Fig. 1, the monitoring apparatus 2 may include: the first interface 21 detachably connected to the monitoring bougie 1, the signal processing module configured to receive the processed signal obtained by processing the collected signal transmitted by the monitoring bougie 1, and the display module configured to display the processed signal. The monitoring bougie 1 may include, as shown in Fig. 2, the sensor 112 configured to collect the collected signal.

The monitoring apparatus 2 may be connected through the first interface 21 to the second interface of the monitoring bougie 1. The connection between the first interface 21 and the second interface 122 may be a plug connection, a clip-on connection or a threaded connection. The signal processing module may perform a series of processing on the collected signal, including weak signal filtering and noise reduction, primary amplification, storage, A/D conversion, post processing and amplification, and so on. Therefore, the signal processing module may include a filter, an amplifier, an A/D converter, a memory, corresponding feedback circuits, etc. The display module may be an LCD display, an OLED display, and the like.

According to the above description, since the monitoring bougie 1 and the monitoring apparatus 2 are connected in a detachable manner, during an inspection for a living body, such as CT, MR, or other inspection that affects or is affected by an electronic circuit, the monitoring apparatus 2 may be removed from the monitoring bougie 1 so as to perform the inspection. At that time, the monitoring bougie 1 is powered off, which will not affect the inspection result.

Fig. 2 is a schematic diagram of the connection between the monitoring bougie and the monitoring apparatus according to an embodiment of the present disclosure. Also, Fig. 2 shows an example of the monitoring bougie 1 and the monitoring apparatus 2. Fig. 2 shows an example of the rear end of the monitoring bougie 1 connecting the socket F (first interface 21) of the monitoring apparatus 2 via the first wire 13 and the plug E (second interface 122) at an end thereof. The first wire 13 may be a wire coated by a layer of insulating materials and transfers the collected signal. E is a detachable plug or a contact device in other forms and connectable with the socket F of the monitoring apparatus 2. As shown above, the monitoring bougie 1 is partly embedded in a body, the end of the plug E side is exposed to the outside of the living body, and the monitoring bougie 1 is connected to the socket of the monitoring apparatus 2 via the plug E. The monitoring bougie 1 is capable of collecting intracorporal parameters to obtain the collected signal and transferring the collected signal through the first wire 13, the plug E and the socket F to the monitoring apparatus 2. The monitoring apparatus 2 may receive the collected signal via the receiving module (not shown).

As shown in Fig. 2, the monitoring apparatus 2 may further include a power module, a power switch G, a display K, and warning lamps H, I, and J. The display K may display the contents including the value of the monitored parameter based on the collected signal (for example, the values of the patient's intracranial physiological parameters), and also display a power level, the remaining service time, the wired/wireless connection status, the time of implantation of the bougie, the warning status, the warning elimination/cancellation indicator, technical warning indicator, waveforms of the processed signal, etc. The warning lamps H, I and J respectively warns about battery level, a wireless connection signal and monitoring parameter values. The warning lamps and the warning contents include but are not limited hereto.

In a possible embodiment, the monitoring apparatus 2 further comprises a first warning module configured to generate a warning signal to issue a warning according to the relationship between the processed signal and the warning threshold value. For example, the warning threshold value of the monitored parameters can be preset. The signal processing module in the monitoring apparatus 2 processes the signal to obtain the processed signal, compares the processed signal with the warning threshold value, and generates a warning signal according to the comparison result and issues a warning. The warning may be issued by the afore-described warning lamps or by sound, or in a combination thereof, which is not limited. Hence, the medical personnel can be timely informed of accidents occurred during the monitoring process and take actions immediately.

In a possible embodiment, the first warning module may issue no warning when communication connection (including wired or wireless communication connection) is established between the first terminal and the monitoring apparatus, for example, by disabling the first warning module. The first terminal may include a second warning module. The second warning module may, when communication connection (including wired or wireless communication connection) is established between the first terminal and the monitoring apparatus, generate a warning signal to issue a warning according to the relationship between the processed signal and the warning threshold value, or issue a warning according to the warning signal issued by the monitoring apparatus. Thus, the electric power to the monitoring apparatus 2 is saved. The first terminal may be an apparatus capable of synchronizing the collected signal or further processing and displaying the collected signal, for example, a monitor, an upper computer, and the like.

The power module is a primary, interchangeable or chargeable (detachable or non-detachable) battery that provides stable operation voltage and current and continuously provides power for the monitoring bougie 1 and the monitoring apparatus 2. It has the characteristics of high capacity and small volume. The chargeable battery may be charged by a power adapter or a mobile power source, or may be charged when connecting to the first terminal. Both the monitoring bougie 1 and the monitoring apparatus 2 are featured by low power consumption, relatively high operation speed, and small volume, and are formed by highly integrated circuit, etc.

Fig. 3 is a block diagram of the monitoring apparatus 2 according to an embodiment of the present disclosure.

In a possible embodiment, the monitoring apparatus 2 further comprises either or both of the wired communication module 22 capable of wired communication with the first terminal 3 and the first wireless communication module 23 capable of wireless communication with the first terminal 3.

In a possible embodiment, communication connection may be established between the monitoring apparatus and the first terminal 3in a broadcast or handshake manner.

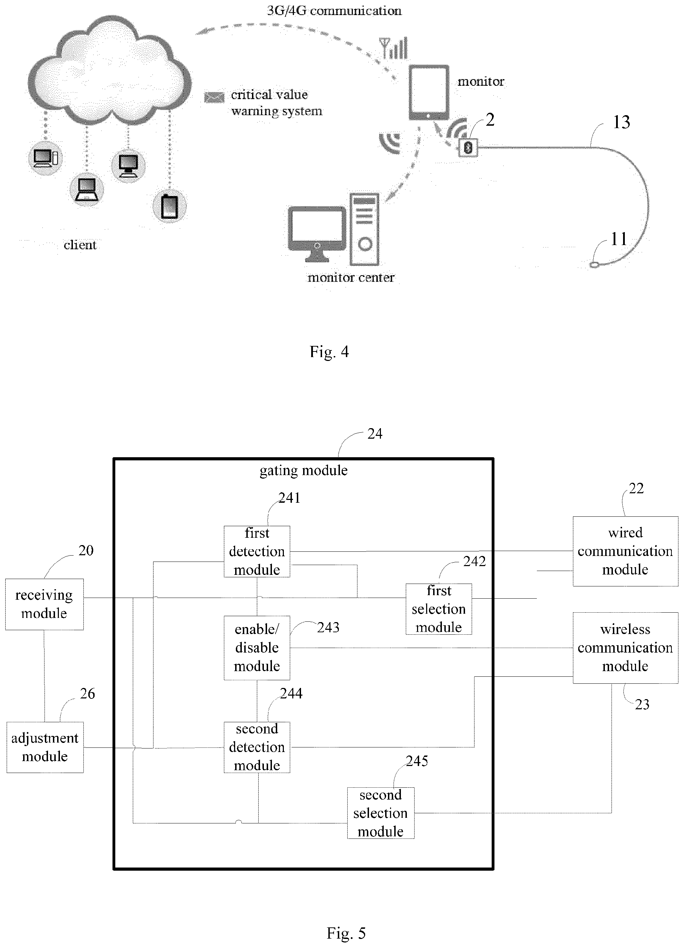

Fig. 4 is a schematic diagram of the monitoring system according to an embodiment of the present disclosure. As shown in Fig. 4, the first terminal 3 may be an apparatus capable of synchronizing the collected signal or further processing and displaying the collected signal, for example, a monitor, an upper computer, a monitor center, and the like.

The wired communication module 22 may connect to the first terminal 3 through a communication cable (data wire, network wire, optical fiber, etc.) and transmit collected signal in a wired manner. The communication cable is connected to the monitoring apparatus 2 and/or the first terminal 3 by a detachable plug, for example, an interface (e.g., USB, RS232, or RS485). Thus, the monitoring apparatus 2 and the first terminal 3 are detachably connected. In other words, the wired communication module may be realized by a hardware such as a data interface (not limited to a data interface; the wired communication module may also be a communication interface and the like), and is capable of transferring data in the manner of wired communication after data processing such as data compression, protocol encapsulation, and so on. The detachable plug may use an aviation plug, a Type C plug, a USB plug, or a lightning plug.

The first wireless communication module 23 may be one or more of a Bluetooth communication module, a WiFi module or an IR transmission module, configured to communicate with the first terminal 3 in a wireless manner. The first terminal 3 may include a module corresponding to the first wireless communication module 23 to receive data transmitted by the first wireless communication module 23. Within a certain spatial domain, the first wireless communication module 23 is capable of accurate and continuous signal exchange with the first terminal.

It should be noted that the afore-described wired communication module 22 and first wireless communication module 23 may be realized by the existing wired or wireless communication technology.

As shown in Fig. 3, in a possible embodiment, the monitoring apparatus 2 may further include a receiving module 20 configured to receive the collected signal transmitted by the monitoring bougie 1, and a gating module 24 connected to the receiving module 20, the wired communication module 22 and the first wireless communication module 23, and configured to transfer the collected signal received by the receiving module 20 to one of the wired communication module 22 and the first wireless communication module 23, so as to transmit the collected signal to the first terminal 3.

The gating module 24 is capable of monitoring the communication connection status of the wired communication module 22 and the first wireless communication module 23, and transmitting the collected signal to one of the wired communication module 22 and the first wireless communication module 23 according to the monitoring result, so as to transmit the collected signal to the first terminal 3. The gating module 24 may also be realized by hardware. For example, the gating connection can be realized by a plurality of switches controlled by high and low electric levels, or other methods, which is not limited in the present disclosure.

The monitoring apparatus in the related art usually uses a wire to connect the monitoring bougie with an extracorporeal device, which is suitable for transferring a large amount of data. But the wire length is limited, which limits the range of motion of the monitored object; and when used for a monitored object with poor compliance (such as a patient with disturbance of consciousness, or an experimental animal that does not cooperate), the measuring wires are easily removed from the body by mistake, or cannot stably and accurately monitor the relevant parameters. In order to achieve the monitoring performance, the traditional monitoring apparatus requires system integrity, that is, the system must comprise a sensor, a connection cable, a signal processing section and a display (monitor), and must be complete. Therefore, in some cases (such as during patient transport), the limitations of objective conditions lead to the absence of the extracorporeal device (monitor), and it is not possible to monitor physiological parameters in a timely and effective manner, which significantly affects the clinical diagnosis and animal experiments, even causes life-threatening accidents in severe cases.

The monitoring apparatus 2 according to the present disclosure may transmit the collected signal in one of a wired connection and a wireless connection, and overcomes the problem of the range of the wired connection and the system integrity. The transmission method can be chosen according to various application scenarios as appropriate. Thus, the monitoring apparatus 2 according to the present disclosure is suitable for a plurality of monitoring scenarios.

Fig. 5 is a block diagram of the monitoring apparatus according to an embodiment of the present disclosure. As shown in Fig. 5, in a possible embodiment, the gating module 24 may further include the first detection module 241 configured to detect whether the wired communication connection between the wired communication module 22 and the first terminal 3 is normal; and the first selection module 242 connected to the first detection module 241 and configured to, if the first detection module 241 detects that the wired communication connection between the wired communication module 22 and the first terminal 3 is normal, transfer the collected signal to the wired communication module 22.

The first detection module 241 is capable of transmitting a simple test signal to the first terminal 3 through the wired communication module 22 and determining whether the wired communication connection is normal according to the feedback of the first terminal 3. Alternatively, the first terminal 3 may regularly transmit a signal to the wired communication module 22, and the first detection module 241 determines whether the wired communication connection is normal by detecting whether the wired communication module 22 receives the signal. For example, the first detection module 241 transmits a simple test signal to the first terminal 3 through the wired communication module 22. Under the condition that the first terminal 3 feeds back a signal of successful connection, it is determined that the wired communication connection is normal. Alternatively, whether the wired communication connection is normal is detected by handshake based on a communications protocol. The above two detection methods can both be realized based on the existing hardware communications technology.

When the wired communication connection is normal, the first detection module 241 transmit the detection result to the first selection module 242 in form of a command. The first selection module 242 may transmit the collected signal to the wired communication module 22 according to the received detection result, so as to transmit the collected signal to the first terminal 3 in the manner of wired communication. If the feedback signal of the first terminal 3 is not received, it is determined that the wired communication connection is abnormal, in which case the collected signal will not be transferred by wired communication, and may be transferred by wireless communication or directly stored. The first selection module 242 may be realized by a switch. If the wired communication connection is normal, the wired communication is preferred, so as to more rapidly and accurately transfer the collected signal with a high efficiency.

In a possible embodiment, if the first detection module 241 detects that the wired communication connection between the wired communication module 22 and the first terminal 3 is normal, the first wireless communication module 23 may be disabled. It is possible to reduce the power consumption of the system by keeping the first wireless communication module 23 disable when the wired communication connection is normal.

In a possible embodiment, the gating module 24 may further include an enable/disable module 243 connected to the first detection module 241 and configured to enable the wireless communication module 23 and transmit a command to the second detection module 244 if the first detection module 241 detects that wired communication connection between the wired communication module 22 and the first terminal 3 is abnormal, and a second detection module 244 connected to the enable/disable module 243 and configured to, in response to the command from the enable/disable module 243, detect whether the wireless communication connection between the wireless communication module 23 and the first terminal 3 is normal.

If the first detection module 241 detects that the wired communication connection is abnormal, it may transmit to the enable/disable module 243 an enable command to enable the wireless communication module 23. When receiving the enable command, the enable/disable module 243 generates an enable control signal and transmits the enable control signal to the wireless communication module 23 to enable the wireless communication module 23, and generates a detection command and transmits the detection command to the second detection module 244. The second detection module 244 detects whether the wireless communication connection between the first wireless communication module 23 and the first terminal 3 is normal according to the detection command.

The modules described above may each be realized in one or more forms by means of software or hardware. For example, the enable/disable module 23 may be realized by a switch, of which the connection and disconnection realizes the connection and disconnection between the wireless communication module 23 and the power source, so as to control enabling and disabling the wireless communication module.

For example, the first wireless communication module 23 may be one or more of the Bluetooth communication module, the WiFi or the IR transmission module according to the prior art. Each of these modules contains a corresponding signal generator. By controlling the signal generator in the first wireless communication module 23, the second detection module 244 is capable of transmitting a request signal for wireless connection to the first terminal 3 to request for establishing wireless communication. The first wireless communication module 23 may feed back to the second detection module 244 a signal of the connection status, for example, whether the connection is successful, the signal quality, etc. The above can be realized by the existing wireless communication technology.

In a possible embodiment, the gating module 24 further includes a second selection module 245 connected to the second detection module 244 and configured to transfer the collected signal to the first wireless communication module 23 if the second detection module 244 detects that the wireless communication connection between the first wireless communication module 23 and the first terminal 3 is normal.

If the second detection module 244 detects that the wireless communication connection between the first wireless communication module 23 and the first terminal 3 is normal, it may transmit a command to the second selection module 245. When receiving the command, the second selection module 245 transmits the collected signal to the first wireless communication module 23 according to the command. The second detection module 244 may be realized by hardware, for example, by a switch.

In the monitoring apparatus 2 according to the afore-described embodiment of the present disclosure, the gating module 24 is capable of detecting the connection status of the wired communication and the wireless communication and transferring the collected signal in a proper communications method. When the medical personnel has to leave the monitored object temporarily, or under the condition that the space is insufficient, the first terminal 3 may be detached from the monitoring apparatus 2, and a connection is established by the wireless communication module, so that the monitoring system is no longer limited by the space. In general cases, the collected signal is transferred by wired communication, which is favorable for transferring a large amount of high-quality data.

In a possible embodiment, if the second detection module 244 detects that the wireless communication connection between the first wireless communication module 23 and the first terminal 3 is abnormal, the first wireless communication module 23 is disabled, which makes it possible to reduce the power consumption of the monitoring apparatus.

In a possible embodiment, if the second detection module 244 detects that the wireless communication connection between the first wireless communication module 23 and the first terminal 3 is abnormal, the enable/disable module 243 disables the first wireless communication module 23, and regularly enables the first wireless communication module 23 so as to try to establish a connection with the first terminal 3. Meanwhile, the first detection module 241 may constantly detect whether the wired communication connection between the wired communication module 22 and the first terminal 3 is normal by the afore-described method. Data can be transmitted to the first terminal 3 when either of the wired communication and the wireless communication connection restores.

For example, under the conditions that the first detection module 241 detects the wired communication connection between the wired communication module 22 and the first terminal 3 to be abnormal and that the second detection module 244 detects the wireless communication connection between the first wireless communication module 23 and the first terminal 3 to be abnormal, the enable/disable module 243 may disable the first wireless communication module 23. In order to timely establish a connection and transfer data when the communication restores, the first wireless communication module 23 may be enabled every 1 minute to try connecting the first terminal 3. If the connection is still disabled, the first wireless communication module 23 is disabled. For example, the enable/disable module 243 may be controlled by a timer to enable the first wireless communication module 23.

It should be noted that, although the above example is described with an interval of 1 minute, the afore-described monitoring apparatus is merely exemplary. A person skilled in the art understands that the present disclosure is not limited thereto. In fact, the user can set the cycle period of enabling and disabling the first wireless communication module 23 flexibly according to personal preference and/or the actual application scenario, as long as it is possible to timely establish the connection.

In a possible embodiment, the monitoring apparatus 2 may further include an adjustment module 26 connected to the first detection module 241, the second detection module 244, and the receiving module 20, and configured to cause the receiving module 20 to sample the collected signal at a first sampling frequency if the first detection module 241 detects that the wired communication connection between the wired communication module 22 and the first terminal 3 is normal, and configured to cause the receiving module 20 to sample the collected signal at a second sampling frequency if the first detection module 241 detects that the wired communication connection between the wired communication module 22 and the first terminal 3 is abnormal and the second detection module 244 detects that the wireless communication connection between the first wireless communication module 23 and the first terminal 3 is normal. The first sampling frequency is higher than the second sampling frequency.

When the wired communication connection is normal, the transmission rate data is relatively high. Thus, sampling the collected signal at a relatively high sampling frequency may ensure a relatively high data quality. When the wired communication connection is abnormal while the wireless communication connection is normal, the transmission rate data is relatively low in consideration of the data quality and the transmission rate. In order to avoid distortion when the sampled signal restores, the second sampling frequency cannot be excessively low (for example, the Nyquist Sampling Theorem needs to be satisfied).

The adjustment module 26 can be realized by hardware. For example, the adjustment module is capable of switching sampling pulses at various frequencies to the receiving module 20 according to the command from the first detection module 241 and second detection module 244, so as to alter the sampling frequency of the receiving module.

Hence, when the monitoring apparatus according to the afore-described disclosure transfers data by wireless communication, the input collected signal is sampled at a relatively low sampling frequency and is then transmitted by the wireless communication module 23, so as to ensure the efficiency and quality of the transmission. When the data is transferred by wired communication, the input collected signal is sampled at a relatively high sampling frequency and is then transmitted by the wired communication module 22, preferably transferred with relatively high data quality. Thus, the monitoring apparatus is capable of transmission in a wired manner with high data quality, if allowed by the spatial and other conditions, and, under limited conditions, is capable of wireless transmission, giving due consideration to the transmission efficiency and the data quality, thereby overcoming the defect of each of the wireless transmission and the wired transmission.

The afore-described modules including the adjustment module and the enable/disable module can all be realized by hardware, for example, by a special integrated circuit, a circuit formed by discrete elements, a processor capable of executing logical instructions, a single chip microcomputer, or a field programmable logic device, and so on.

In a possible embodiment, the first terminal 3 may comprises a wired receiving module and a wireless receiving module corresponding to the wired communication module and the first wireless communication module, respectively. For example, the wired receiving module may be a wired communication interface corresponding to the wired communication module; the wireless receiving module may be one or more of a Bluetooth communication module, a WiFi signal receiving module and an IR receiving module. The first terminal may be an external monitor and is capable of further processing and displaying the collected signal.

In a possible embodiment, the monitoring apparatus 2 further comprises a first storage module configured to store the processed signal when communication with the first terminal 3 is interrupted. The first storage module may be a memory chip, a flash disk, an SD memory card, a TF card, etc. and allows read/copy the monitoring data by taking out the data card. When communication with the first terminal 3 is interrupted, the monitoring apparatus 2 is capable of storing the monitored parameter data in a first storage module. If detecting restoration of the connection with the first terminal 3, the monitoring apparatus 2 transfers the stored data to the first terminal 3.

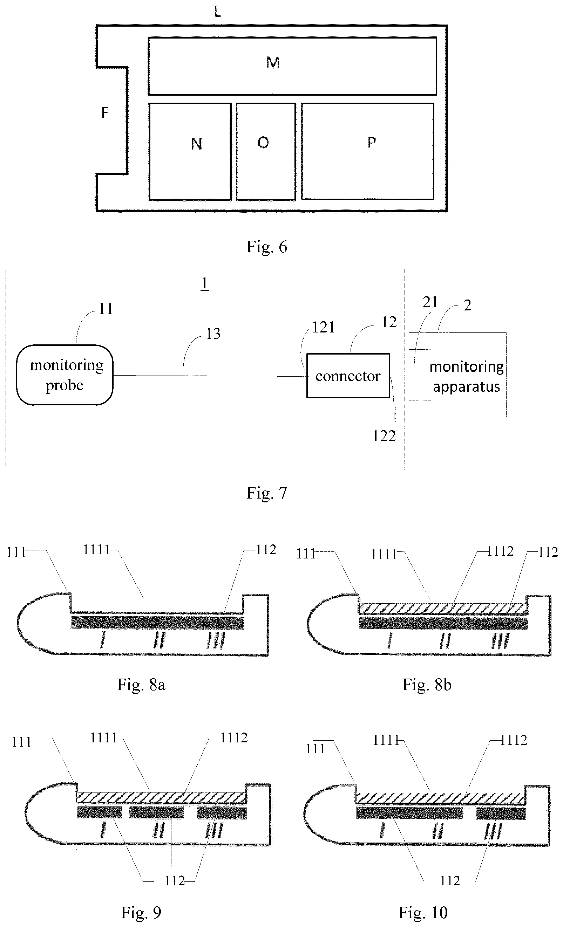

Fig. 6 is a schematic diagram of the monitoring apparatus 2 according to an embodiment of the present disclosure. As shown in Fig. 6, F is a socket (the first interface) connected with the wire plug E with certain fastness. For this purpose, the socket and the wire plug may be configured to have an additional device such as a buckle to secure the connection between the wire plug and the socket and prevent pulling out by mistake. The socket needs to satisfy the conditions of low resistance, high sensitivity, low interference, and so on, to reduce the interference of various noises with the signal generated by the sensor and transferred through the wire and to improve the sensitivity and accuracy of the monitoring. L may be a housing of the monitoring apparatus 2 which has an insulating performance and is sealed. M may be the power module 5, which is a primary, interchangeable or rechargeable battery for providing a stable operation voltage and current as well as providing power for other components. N may be a data processing module (including the receiving module) which performs processing such as amplification, filtration and noise reduction, A/D conversion and sampling frequency setting for the received data collected by the sensor, performs processing such as amplifying and analyzing the A/D converted signal as required, compares the result with a set range of warning value and with a proximate sampled value to determine the accuracy and the reliability of the data and to determine whether to issue a warning, and transmits the warning signal to the warning system, and is also capable of sampling the data at a particular frequency and transmitting the data to the wireless/wired transmission module (the wireless/wired communication module) to transfer the date. O can be a first storage module configured for data buffering and storing the monitored value obtained in a certain period, so as to facilitate collected signal buffering when both the wired and the wireless connection are interrupted. P can be a wired/wireless transmission module (wireless/wired communication module) configured to transmit the processed (encoded, compressed, etc.) data to the first terminal 3 and capable of receiving an instruction from an external apparatus (including the first terminal) to set the data processing module, and so on. There may further be comprised a power management module (not shown) and an peripheral circuit for monitoring the operation voltage and current and the battery level of the power source M, and transmitting the warning signal to the warning system when the voltage, the current, or the battery level is lower than a preset value (usually slightly greater than a rated value for normal operation).

The monitoring apparatus according to the present disclosure may form a simple and independent monitoring system to perform physiological parameter monitoring and data collection, recording, analysis, display, indication, and has a sound and/or light warning function. Such a monitoring system is no longer restricted by a monitor; the monitoring apparatus is capable of operating independently as a monitor. When the spatial condition is limited or when the patient is being transported, the monitoring and warning function can be realized without the monitor.

In a possible embodiment, the monitoring apparatus 2 further comprises a control panel for enabling an operator to set zero setting data, patient information, an operation mode, a warning threshold value, warning cancellation/delay, and so on. The control panel may be a physical control panel located on the outer surface of the monitoring apparatus 2 or a touch control panel displayed by the display K.

For example, as shown in Fig. 2, just like the power switch G, a plurality of buttons can be provided on the surface of the monitoring apparatus 2. In a possible embodiment, an operation mode selector switch can be included for controlling the communication method between monitoring apparatus and the first terminal. The operation mode selector switch may be configured to have three positions, including an ON position (simultaneously enabling the wired and the wireless modes), a wired connection position (disabling the wireless mode), and an OFF position (disabling the wired and the wireless transmission). Thus, an observer can control the communication method between the monitoring apparatus 2 and the first terminal 3 by means of the operation mode selector switch. The operation mode selector switch can be a single switch that shifts to a different position by each pressing. Alternatively, it can include a plurality of sub-switches, so as to shift to a different position by pressing a different sub-switch.

In addition, a special processing module may be integrated within the monitoring apparatus 2. The processing module controls the communication method by identifying the operation on the operation mode selector switch. For example, when the observer selects the OFF position, the processing module receives the processed signal, so as to control to disable the wired communication module and the first wireless communication module. Thus, when the apparatus has a low battery level or when there is no terminal, the observer may select the OFF position to reduce the power consumption. At that time, the collected signal may be stored in the first storage module. When the observer selects the wired connection position, the processing module controls to enable the wired communication module to detect whether the wired communication connection is normal; if yes, data is transmitted by wired communication; if the connection is abnormal, the monitoring apparatus 2 may provide a warning signal to notify the observer. When the observer selects the ON position, the processing module controls to enable the wired communication module and the first wireless communication module to detect whether the wired communication connection is normal; if yes, the signal is transferred by wired communication; if the wired connection is abnormal, it is detected whether the wireless communication connection is normal; if the wireless communication connection is normal, the signal is transferred by wireless communication; if the wireless communication connection is abnormal, the monitoring apparatus 2 may provide a warning signal to notify the observer; alternatively, under this condition, the wireless communication is automatically disabled, and automatically enabled to try the connection during a certain period of time. The above is an example of the present disclosure. The above process may also be realized by a plurality of modules (e.g., the enable/disable module, the detection module, the gating module, and the warning module), respectively.

In a possible embodiment, the monitoring apparatus 2 further comprises a switch button, a threshold value setting button, a warning cancellation/delay switch, a zero setting button, a menu setting button, a record review button, a wireless connection button, a display awaking button, etc. The switch button is capable of controlling turning on and off the monitoring apparatus 2. The threshold value setting button allows setting the warning threshold value. The warning cancellation/delay switch allows controlling to cancel/delay displaying the warning signal. The zero setting button allows zero setting the monitoring bougie 1. The menu setting button allows setting the display menu of the display K. The record review button allows inquiries of history memory. The wireless connection button allows controlling to enable and disable the wireless communication module. The display awaking button allows controlling the lighting and extinction of the display K. The above buttons may be push buttons or may be realized by a touching a touch panel provided on the monitoring apparatus 2. Alternatively, the display K may be a touch screen and displays the control panel.

In a possible embodiment, a voice input module and a voice button may be further provided on the monitoring apparatus 2 or the first terminal 3. Voice can be recorded by means of the voice input module and the voice button. The recorded voice can be inquired and displayed, retrieved, etc. in history record data/curve.

In a possible embodiment, an instruction transmitted by the first terminal 3 may be received by the wired/wireless communication module in the monitoring apparatus 2, so as to set the internal parameters of the monitoring apparatus 2. When the monitoring bougie 1, the monitoring apparatus 2, and the first terminal 3 are interconnected, by means of the first terminal 3 and/or the monitoring apparatus 2, it is possible to set zero, set the warning threshold value, input patient information and monitoring records, etc. The memory chip of the monitoring bougie 1, the monitoring apparatus 2 and the first terminal 3 record data at the same time, which facilitates retrieval as needed. When the first terminal 3 is removed, the system formed by the monitoring apparatus 2 and the monitoring bougie 1 is capable of performing the monitoring function independently.

In a possible embodiment, the monitoring apparatus 2 has a dimension of a length less than or equal to 7 cm, a width less than or equal to 5 cm, and a thickness less than or equal to 2 cm. As shown in Fig. 4, the monitoring apparatus 2 may be a miniaturized electronic circuit apparatus that is portable and attached to an exterior of the monitored object at a position on the external surgical dressings and clothing, etc., having no influence to the motility of the monitored object. The monitoring apparatus 2 may include a processor, a display module, and the like, functioning for display and warning after processing the collected signal for the observer to observe. In a possible embodiment, the monitoring apparatus 2 may be wearable and be attached to the head or the body of the monitored object by an attachment accessory (e.g., bandage, clip, etc.). It overcomes the defect of motion space and the integrity requirement of the system, and is applicable to a plurality of monitoring scenarios.

Since the monitoring apparatus 2 is detachably connected to the first terminal 3 and the monitoring apparatus 2 is portably attached to the exterior of the monitored object, when the medical personnel has to leave the monitored object temporarily or when the spatial condition cannot be satisfied, the first terminal 3 can be removed from the monitoring apparatus 2; and the monitoring apparatus 2 is capable of continuing to monitor the intracorporal parameters of the living body. The wireless communication module establishes a connection with the first terminal 3 and transmits data, so that the monitoring system is free from the limitation of the space.

By a detachable connector, the monitoring bougie 1 is connected with a miniaturized electronic circuit (the monitoring apparatus 2) portably attached to the exterior of the monitored body. The electronic circuit processes the collected signal and then functions for display and warning for the observation of the observer. The monitoring apparatus 2 may be removed during an MR, CT or other inspection that affects or affected by an electronic circuit, so as to prevent the electronic circuit in the monitoring apparatus 2 from affecting the inspection result.

As shown in Fig. 4, the monitoring apparatus 2 may transfer the monitoring data to a cloud server or a monitor center and to a client terminal such as a particular computer or a mobile phone in a wireless manner, such as by Wi-Fi, 3G/4G, etc. The observer is thus freed from the spatial limitations and can access the server through the client terminal to know about the monitoring situation at real time. When the monitoring apparatus 2 or the first terminal 3 (e.g., a monitor host, a monitor center) provides a warning signal and transmits the warning signal to a particular terminal in a particular manner (by a message, or a notification to client terminal), so as to warn the observer, without being limited by the space.

As shown in Figs. 3 and 4, the present disclosure further provides a monitoring system further comprising a first terminal 3 configured to receive the processed signal from the monitoring apparatus 2, analyze the processed signal to obtain monitoring data, and display the monitoring data. For example, it may plot points of the processed signal to display the monitoring situation (monitoring waveform) in real time, and may analyze the monitoring waveform according to different principles, for example, with respect to the intracranial pressure, performing a trend analysis, P1/P2/P3 waveform analysis, and predicting a trend of deformation, etc.

The monitor host may be an example of the first terminal described above. Compared to the monitoring apparatus, the monitor host has a greater volume and more comprehensive functions, and thus is suitable for bedside monitoring of the monitored object (the patient). However, a person skilled in the art should understand that the first terminal 3 can be an arbitrary monitor device capable of receiving, analyzing and displaying the processed signal, for example, a monitor center that simultaneously monitors a plurality of monitored objects, a remote control terminal, etc., which is not limited in the present disclosure.

In a possible embodiment, the system may further include a server connecting the first terminal 3 and configured to receive the monitoring data transmitted by the first terminal 3. The server connects the second terminal. The second terminal is capable of acquiring the monitoring data by means of the server.

The server may be a local server or a cloud server and is capable of receiving and storing monitoring data uploaded by the first terminal 3 through the Ethernet, WiFi or 3G/4G, and encrypting the monitoring data as appropriate. The server supports data inquiries performed by clients of a plurality of second terminals. The server supports to distribute the warning data or the prompting message regarding the monitored object, and issues warnings to the clients of a plurality of second terminals in form of message, notification, etc.

The second terminal may be an arbitrary terminal such as PC, mobile phone, pad, etc. A client may be installed in the second terminal. The second terminal may include clients on Windows, iOS, MacOS, Android and other platforms. The second terminal may be in communication with the server in a wired or wireless manner. An observer (e.g., a doctor) may log in the server on the client of the second terminal using an account and password to check the real time monitoring data and history data of the associated monitoring bougie and set the monitoring apparatus and the monitor host.

The monitoring system according to the embodiments of the present disclosure is capable of acquiring the collected signal from the monitoring apparatus and processing the signal. The signal is further analyzed and displayed by the first terminal which transfers the analyzed monitoring data to the server. The monitoring data can be viewed on the terminal. Thus, the monitoring system according to the embodiments of the present disclosure realizes remote reception and processing of the physiological parameter monitoring data, so as to monitor the physiological parameters effectively in real time.

The present disclosure further provides a monitoring bougie 1. Fig. 7 is a schematic diagram of the monitoring bougie according to an embodiment of the present disclosure. As shown in Fig. 7, the monitoring bougie 1 comprises: a monitoring probe 11 configured to acquire the collected signal; a connector 12, one end 121 of which connects to the monitoring probe 11 via a first wire 13 and one other end provided with a second interface 122 detachably connectable to the monitoring apparatus 2; and a second storage module (not shown) configured to store an ID of the monitoring bougie and a preset threshold value.

In a possible embodiment, the monitoring probe 11 includes a sensor 112 configured to collect the collected signal. The monitoring probe 11 may include one or more sensors of different technologies for monitoring one or more target physiological parameters, such as an intracranial pressure sensor, a temperature sensor, etc. The sensor may be physically insulated from the intracorporal environment by a sensor external protection device. This insulation does not apparently affect the collection of the target physiological parameters or has an effect that can be eliminated by subsequent processing. That is, the insulated and/or subsequently processed sensor has high stability and high sensitivity, and thus can accurately monitor the target physiological parameters.

As shown in Fig. 2, the sensor 112 is physically insulated from the intracorporal environment by the sensor external protection device (housing) 111. The housing 111 may be a metallic or a polymer sensor protection device and also a base for mounting the sensor. It may have a window which facilitates the sensor to collect data more directly. The sensor 112 may be covered by a packaging layer 1112 serving to insulate the circuit elements from the intracorporal environment without affecting the collection of physiological parameters.

The sensor and the sensor external protection device form the monitoring probe 11. The monitoring probe 11 can be embedded in the body of a living body. The connector 12 and a part of the first wire 13 may be exposed to the outside of the body, so as to connect the monitoring apparatus 2.

In a possible embodiment, the sensor may be a piezoelectric or piezoresistive sensor. The circuit of the sensor may be in a full-bridge or half-bridge connection. The sensor is capable of measuring an absolute pressure or a relative pressure.

In a possible embodiment, the monitoring probe has a diameter that is smaller than or equal to 800µm, for example, 600µm.

In a possible embodiment, the second interface 122 may have an arbitrary form of a pluggable connection, a clip-on connection or a threaded connection. The monitoring apparatus 2 has a first interface 21 corresponding to the second interface 122. Taking the pluggable connection as an example, the second interface 122 may be a male interface while the first interface 21 may be a female interface; alternatively, the second interface 122 may be a female interface while the first interface 21 may be a male interface. The second interface 122 and first interface 21 may be use an aviation plug, a Type C plug, a USB plug, or a lightning plug, etc. The communication between the second interface 122 and first interface 21 may be based on the existing wired communication protocols to transfer the collected signal. The connector 12 connects the monitoring apparatus 2 through the second interface 122, so that the monitoring apparatus 2 is detachable from the connector 12.

The monitoring probe 11, the connector 12 and the first wire 13 may be packaged to form the monitoring bougie 1. By detachably connecting the monitoring bougie 1 and the monitoring apparatus 2, the monitoring apparatus 2 is removable in an MR, CT or other inspection that affects or is affected by electronic circuits, so as to prevent the electronic circuits in the monitoring apparatus 2 affect the inspection result, as well as to prevent the monitoring apparatus 2 from injuring the user due to electromagnetic interference. Moreover, the monitoring probe 11 is placed inside the body of the user, so as to avoid the miscellaneous operation of plugging the monitoring probe 11 and/or secondary injury to the user caused by repeated surgical implantation.

The second storage module may be a memory chip, for example, a memory chip realized by ASIC (Application Specific Integrated Circuit) or FPGA technology, and is configured to store the ID of the monitoring bougie and the preset threshold value. The second storage module is also capable of storing the zero setting data, the threshold value of warning in the monitoring, patient information, the time of implantation, the factory version of the monitoring bougie, the collected signal (e.g., the intracranial physiological parameters), etc. The zero setting data allows the use of the monitoring bougie 1 even when the monitoring apparatus 2 is replaced. The monitoring bougie 1 is adapted to a plurality of monitoring apparatuses 2 and does not need further zero setting. The medical personnel may add patient information such as user information and other information to the bougie, so as to reserve the patient information such as the use record.

By configuring the second storage module on the monitoring bougie 1, it is possible to back up various data, so as to realize the independency of the monitoring bougie 1 and the monitoring apparatus 2. When the monitoring bougie 1 is removed from the monitoring apparatus 2, it is connectable with a different monitoring apparatus 2. The monitoring apparatus 2 is capable of continuing the monitoring operation by identifying information (the ID of the monitoring bougie) recorded in the memory chip of the monitoring bougie 1.

The second storage module may store a preset threshold value (e.g., the warning threshold value), so that there is no need to further set the warning threshold value when use. When the threshold value needs to be adjusted, a new threshold value can be set by the monitoring apparatus 2 and stored by the second storage module.

The monitoring bougie 1 is capable of independently storing information (e.g., the collected signal, the patient information, etc.), so that the monitoring data is safer and will not be lost due to the damage, loss, or exchange of the external monitoring apparatus 2/terminal or due to false operation of the user.

In a possible embodiment, the second storage module may be a memory chip that is not affected by an MR, CT or other inspection affecting electronic circuits, which further guarantees the data security. For example, the second storage module may be a ferroelectric memory which has strong anti-interference and of which the information storage is uneasily subjected to external influences. Hence, the influence on the MR and CT inspection results is further reduced.

In a possible embodiment, the second storage module may be located in the monitoring probe 11, in the connector 12, or between the monitoring probe 11 and the connector 12. For example, a cavity for placing the second storage module may be provided in the protection device of the monitoring probe 11. The second storage module is connectable to each sensor in the monitoring probe 11, so as to store the collected signal acquired by the sensor. Alternatively, the second storage module may be provided in the connector 12 and connect each sensor in the detection probe through a wire.

In a possible embodiment, the monitoring bougie 1 is provided with an electronic identity tag which has uniqueness. The electronic identity tag may be a magnetic strip, a two-dimensional code, etc. Other terminals may obtain through the electronic identity tag the information of the monitoring bougie, such as the preset threshold value, the factory version, the serial number of the sensor, the date of manufacture, the manufacturer, the batch number, etc., and, based on the electronic identity tag, acquire on a network the corresponding information of the monitoring bougie. For example, by means of the electronic identity tag, the server is connected; thus, information of the patient status, the time of implantation can be acquired according to the information uploaded by the monitor/monitoring apparatus 2 and stored by the server.

Figs. 8a and 8b are structural charts of the monitoring probe 11 according to an embodiment of the present disclosure. The monitoring probe 11 is applicable for detection of physiological parameters. As shown in Fig. 8a, the monitoring probe 11 comprises a housing 111, on the surface of which a window 1111 is opened, and a sensor 112 provided at a position facing the window 1111.

The housing 111 is capable of supporting and protecting the sensor 112. The housing 111 may use a material having certain strength. The material is harmless to human body, placeable in the intracorporal environment, and is stable in property and will not have chemical reaction to the fluids in the body. Moreover, corresponding characteristics of the material may be required according to the measurement objective. For example, a material having good thermal conductivity may be used for measuring the brain temperature. The housing 111 can be made of materials including metal, ceramics, polymers satisfying these characteristics. The material of the housing 111 is not limited in the present disclosure.

The housing 111 may be cylindrical shaped (as shown in Fig. 8a) so as to easily insert into the interior of a tissue such as the blood vessel. The housing 111 may also have any shape as required. For example, it may be flat structured. The shape of the housing 111 is not limited in the present disclosure. Moreover, the surface of the housing 111 may be provided to have a flat and smooth structure without apparent sharp edges or corners, so as to avoid injuring the surrounding tissue during the implantation or removal.

The window 1111 is opened in the surface of the housing 111, and the sensor 112 is provided at a position facing the window 1111, so as to allow the sensor 112 detect the intracorporal parameters. The sensor 112 and the window 1111 may be in one-to-one correspondence (e.g., each of a plurality of sensors corresponds to respective window 1111). Or, a plurality of sensors 112 may correspond to one window 1111. Or, one sensor 112 may correspond to a plurality of windows 1111. The window 1111 may have a rectangular shape, a circular shape, an elliptical shape, etc., which is not limited in the present disclosure as long as the sensor 112 is enabled to normal detection of the intracorporal parameters. The signal detected by the sensor 112 may be transferred in a wired or wireless manner to an external processing apparatus, which is not limited in the present disclosure.

In a possible embodiment, the sensor 112 may be provided in the housing 111, at the window 1111 (as shown in Fig. 8a), or directly on the outer surface of the housing 111.

In a possible embodiment, the sensor 112 may be miniaturized; the material of the sensor 112 and the measuring method are harmless to the human body; and the measuring range and accuracy satisfy the actual needs. The sensor 112 may include, not limited to, a sensor for detecting an intracranial pressure intensity which is realized based on MEMS (Micro-Electro-Mechanical System) technology and the like, a sensor for detecting an intracranial temperature by NTC (Negative Temperature Coefficient, indicating thermistor phenomenon and material having negative temperature coefficient in which the resistance exponentially decreases as the temperature increase) or by Thermocouple (a device converting a temperature signal to a thermo-electromotive force signal) method, a sensor for detecting parameters including an intracranial pH value, an oxygen/O₂ content (partial pressure), a carbon dioxide/CO₂ content (partial pressure), a glucose/Glu content, a lactic acid/HL content, and a hemosiderin content by an electrochemical method/fluorescence method. The signal of the sensor may be output by a wire/optical fiber connected to the sensor. The type of the sensor 2 is not limited in the present disclosure.

The sensors may be separate or integrated as one component. Alternatively, a part of the sensors may be separated from one another while a part of the sensors integrated as one component. The integration as one component may include different sensing modules integrated in a single circuit board or a plurality of sensing functions integrated in one circuit, which is not limited in the present disclosure.

In a possible embodiment, as shown in Fig. 8b, a packaging layer 1112 is provided at the window 1111. For example, a sensor realized by an integrated circuit technology such as MEMS or a sensor realized by a circuit is easily affected by the external environment and needs to be packaged and insulated. The packaging layer 1112 provided at the window 1111 may cover the window 1111 and also may cover the surface of the sensor 112, as shown in Fig. 8b. The packaging layer 1112 may directly coat the sensor 112 and then be provided at the window 1111, which is not limited in the present disclosure. The packaging layer 1112 physically and electrically insulates the sensor from the external environment, so as to prevent the structure, the material, and the circuit current of the sensor, and so on, from affecting the intracorporal environment and prevent the adverse effects such as short circuit, chip damage, and so on, caused by liquid leakage, and direct compression of the sensor and the circuit by the intracorporal environment. The material of the packaging layer 1112 may be biologically compatible, which means that the material causes appropriate reaction at particular parts of the body, i.e., is allowed continuous existence in the intracorporal environment. In addition, according to the measurement requirements of the sensor 112, different characteristics may be required for the material of the packaging layer 1112. For example, for a pressure sensor, a certain thickness and elasticity of the packaging layer 1112 is required, so as to well transfer the external pressure to the sensing structure of the sensor; for a temperature sensor, the packaging layer 1112 is required to have certain thermal stability and thermal conductivity, so as to rapidly transfer external heat to the temperature sensor; for a chemical sensor, it is required that the packaging layer 1112 has good permeability of particular molecules, such as O₂, CO₂, HL, Glu, etc., so as to accurately measure the concentration (content). It should be noted that, although the foregoing introduces the required material characteristics of the packaging layer 1112 with the examples of pressure, temperature and chemical molecule detections, a person skilled in the art should understand that the present disclosure is not limited thereto. In fact, the user may flexibly determine the material of the packaging layer 1112 according to the actual application scenarios.

The monitoring probe according to the embodiments of the present disclosure is implantable into the body of a living body (e.g., in the cranial cavity), so as to accurately detect the physiological parameters in real time, which helps in clinical practice to timely and accurately acquire the physiological data and a variation tendency thereof of the patient and facilitates the observation of the development of the disease and the determination of the timing of surgery.

In a possible embodiment, a plurality of sensors 112 may be provided at positions facing the same window 1111. Fig. 9 is a schematic diagram of the monitoring probe according to an embodiment of the present disclosure. As shown in Fig. 9, the plurality of sensors I, II and III may be provided to be separated from one another, with the same or different intervals between each two sensors, which is not limited in the present disclosure. The plurality of sensors 112 are provided to be separated from one another to reduce interference with one another, so as to improve the detection effect and facilitate replacement.

In a possible embodiment, the plurality of sensors 112 are integrated as one component. Figs. 8a and 8b shows an example of the sensors I, II and III being integrated as one component. For example, the plurality of sensors are integrated, including the afore-described sensor for detecting an intracranial pressure and the sensor for detecting an intracranial temperature. Such configuration that plurality of sensors correspond to one window expands the angle of detection. Integrating the plurality of sensors 112 as one component makes it possible to save a process of manufacturing procedure and a process of assembling the detector, and the like.

In a further possible embodiment, the plurality of the sensor 2 may also be provided in the following manner: a part of the plurality of the sensor 112 is integrated together while the rest part is provided separately. Fig. 10 is a schematic diagram of the monitoring probe 11 according to an embodiment of the present disclosure. As shown in Fig. 10, the sensors I and II may be provided to be integrated while the sensor III is provided separately. The sensors can be provided in a different combination. A person skilled in the art can choose the manner of providing the sensors according to the specific application scenario in view of the performances of the sensors.

In the case that a plurality of sensors 112 are provided at positions facing the same window 1111, the packaging layer 1112 may be provided on the entire window. Alternatively, as shown in Figs. 8b, 9 and 10, the packaging layer 1112 may cover a plurality of sensors that are separately provided or a sensor integrated as one component, so as to facilitate the packaging. The packaging layer 1112 may have a one-piece structure satisfying the requirements of the corresponding plurality of sensor or may be patched by different parts satisfying various requirements of the corresponding sensors.

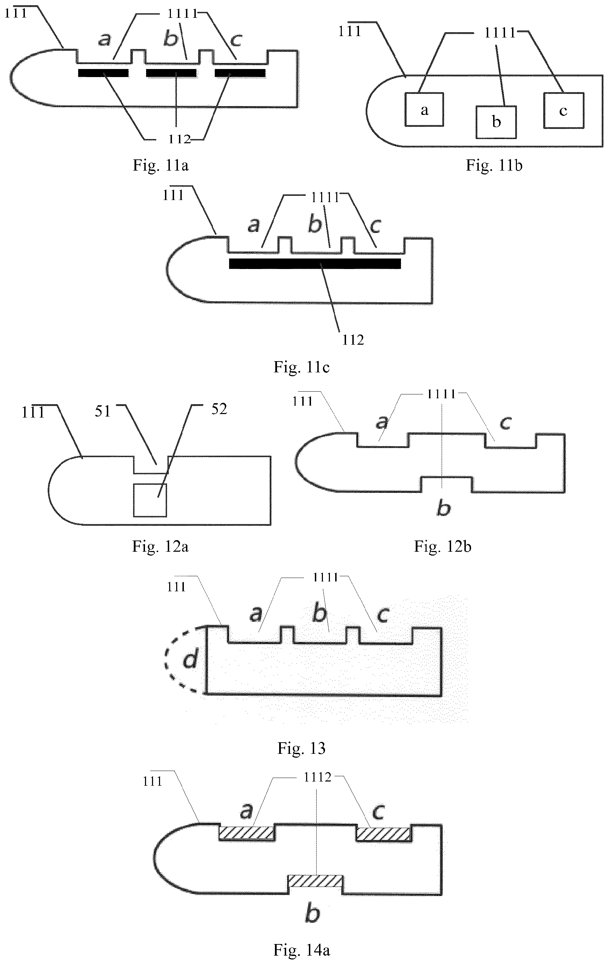

In a possible embodiment, each sensor 112 is provided at a position facing a corresponding window 1111. When there is only one sensor 112, there may be provided only one window 1111, and the sensor 112 being provided at a position facing the window 1111. When there are a plurality of sensors 112, windows 1111 may be provided to be corresponding to each of the sensors 112, the sensors 112 being respectively provided at a position facing the corresponding window, as shown in Fig. 11a.

In a possible embodiment, a window 1111 may be opened in the surface of the housing 111. Figs. 8a, 8b, 9, and 10 show an example where one window is provided.

In a further possible embodiment, a plurality of windows 1111 may be provided in the surface of the housing 111. Figs. 11a to 11c, Figs. 12a to 12b are schematic diagrams of the windows of the monitoring probe 11 according to an embodiment of the present disclosure.

Taking a cylindrical housing 111 as example, as shown in Figs. 11a to 11c, a plurality of windows 1111 may be provided in the side surface of the housing 111. The positions of the plurality of windows 1111 may be on the same side surface of the housing 111. In other words, all of the windows can be completely seen without rotating the housing. Figs. 11a to 11c show different examples with the windows provided on the same side surface. Fig. 11a is a sectional view of the cylinder. Fig. 11b is a top view obtained by viewing downwardly from the side on which the windows are located. As shown in Figs. 11a to 11c, the three windows a, b, and c are arranged in order on the same side surface. The centers of the three windows may be located on the same generatrix on the side surface of the cylinder, as shown in Fig. 11a. The centers of the three windows may also be on different generatrixes in a staggered arrangement, as shown in Fig. 11b. At this time, the plurality of sensors 112 may be separated from one another and located at positions facing the corresponding window 1111, as shown in Fig. 11a. Alternatively, the plurality of sensors 112 may be integrated, as long as the position of each sensor 112 is corresponding to the position of the windows 1111. A cavity is provided below the plurality of windows 1111 for receiving the integrated sensors. The cavity is in communication with the plurality of windows so that the sensors provided in the cavity are capable of detecting the intracorporal parameters through the corresponding windows 1111, as shown in Fig. 11c.

The plurality of windows 1111 may also locate on a plurality of side surfaces. In other words, without rotating the housing, the plurality of windows 1111 cannot all be completely visible at the same time. Figs. 12a and 12b show different arrangements of the windows. In Figs. 12a and 12b, the plurality of windows 1111 locate on a plurality of different side surfaces. As shown in Fig. 12a, the third window 51 is disposed on the upper side surface of the housing 111 while the fourth window 52 locates on the side surface of the housing 111 facing the direction of the paper plane. As shown in Fig. 12a, only a part of the window 51 is visible. Moreover, the third window 51 and the fourth window 52 may be arranged as shown in Fig. 12 to have their centers on the same circumference of the cylinder, or may be in a staggered arrangement, such as on the two ends of the cylinder, which is not limited in the present disclosure. Providing a plurality of windows 1111 on different side surfaces makes it possible to avoid interference among the sensors 112 in the windows and expand the monitoring range and angle. For example, the same sensor 112 may be provided in the windows 1111 on different side surfaces.

In a possible embodiment, the windows 1111 may be disposed on opposing side surfaces of the housing 111. The windows disposed on opposite side surfaces of the housing is in a staggered arrangement, as shown in Fig. 12b, or is disposed to be just opposite to each other, which is not limited in the present disclosure. Disposing the plurality of windows 1111 on opposite side surfaces in a staggered arrangement avoids interference as well as reduces the volume of the monitoring probe 11, which saves the material.

When the plurality of windows 1111 are located on different side surfaces, the plurality of sensors 112 may be integrated as one component, as long as the position of each sensor 112 is corresponding to the position of the window 1111. A cavity is provided below the plurality of windows 1111 (inside the cylinder) for receiving the integrated sensors. The cavity is in communication with the plurality of windows so that the sensors 112 provided in the cavity are capable of detecting the intracorporal parameters through the corresponding windows 1111. Alternatively, each sensor 112 may be provided at a position facing the corresponding window 1111.

In a possible embodiment, the window 1111 may be located on the leading end of the housing 111. Fig. 13 is a schematic diagram of the monitoring probe 11 according to an embodiment of the present disclosure. As shown in Fig. 13, the window d is provided on the leading end of the housing 111. Moreover, the plurality of windows 1111 may be all located on the side surface of the housing 111 or all located on the leading end of the housing 111. Alternatively, a part of the plurality of windows 1111 may be located on the leading end of the housing 111 while one other part located on the side surface of the housing 111. A person skilled in the art may arrange the position of the windows according to the actual needs, which is not limited in the present disclosure. With the window 1111 provided on the leading end of the housing, the sensor 112 placed inside the window 1111 is enabled an expansion of the detection angle to 180 degrees or even more, so as to improve the detection effect.

Arranging the window 1111 at different positions of the housing of the monitoring probe 11 satisfies various requirements of the detection conditions in actual application. Moreover, it is possible to detect a plurality of parameters independently at the same time, without interferences among the detection of each parameter. The comprehensive detection is realizable by implanting only one monitoring probe 11. According to the detected parameters, it is possible to comprehensively determine the development of the disease and the timing of surgery.

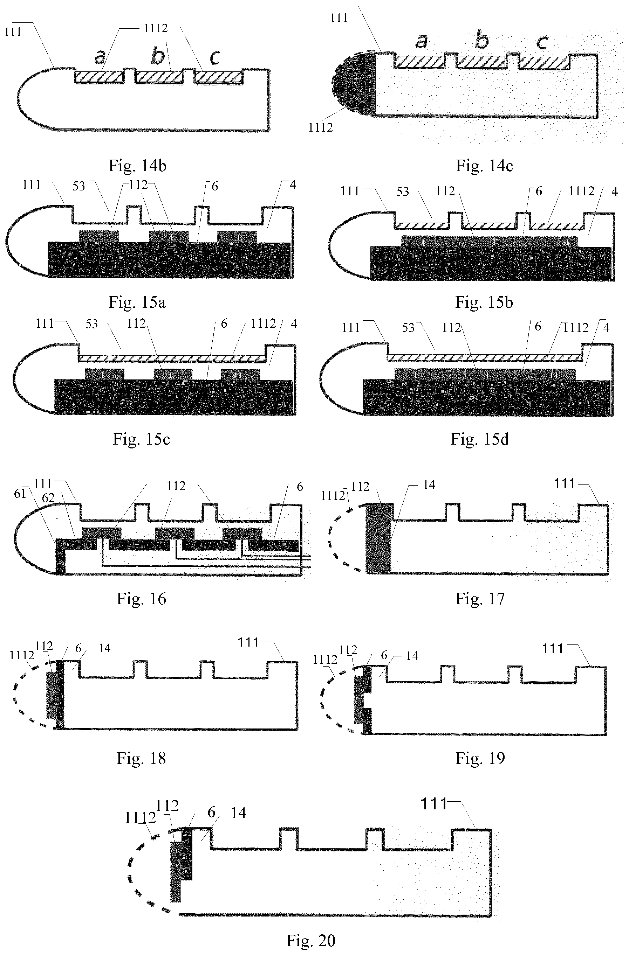

In a possible embodiment, a different packaging layer 1112 is provided at each window at a different position. Figs. 14a to 14c are schematic diagrams of the packaging layer provided according to an embodiment of the present disclosure. As shown in Figs. 14a to 14c, for example, the packaging layer provided at the window 1111 on the side surface may be located inside the window 1111 and cover the sensor 112, or cover the window 1111. Under the condition that side surface of the housing has a plurality of windows 1111, the packaging layer 1112 is provided at each position corresponding to each window 1111, as shown in Figs. 14a and 14b. The packaging layer provided at the window 1111 disposed on the leading end may have a streamline profile or have a surface of smooth transition. Providing the form of the packaging layer 1112 according to different positions makes the shape of the monitoring probe 11 to be more easily implanted into or removed from the body tissue, so as to avoid damaging the surrounding tissue.

Figs. 15a to 15d are structural charts of the monitoring probe 11 according to an embodiment of the present disclosure. As shown in Fig. 15a, the housing 111 is provided therein a hollow cavity in communication with the window.

Corresponding circuits may be laid in the hollow cavity 4 to transfer the signal detected by the sensor 112. In a possible embodiment, the sensor 112 may be provided in the hollow cavity 4 (as shown in Figs. 15a to 15d) or may be directly provided on the outer surface of the housing 111 or at the window.

The sensor 112 is provided on the base 6. The base 6 serves to place, support, and protect the sensor 112 and may use a material of certain strength. The material of the base 6 is harmless to human body, placeable in the intracorporal environment, and is stable in property and does not generate chemical reactions with body fluid. Similarly, the base 6 may be made of a particular metal, ceramics, and a polymer which satisfy the above conditions.

In a possible embodiment, the base 6 and the housing 111 may be formed integrally or formed separately and assembled together, which is not limited in the present disclosure.

In a possible embodiment, as shown in Figs. 15a to 15d, a first window 53 is provided in the side surface of the housing 1. The base 6 is provided in the hollow cavity on a side opposite to the first window 53. The first window 53 may be one or more. The base 3 is provided in association with the position and the number of the first window 53. For example, if only one first window 53 is provided in the side surface of the housing, the base 6 is only provided at a position facing the first window 53. The length of the base 6 may be set according to the length of the window or the dimension of the sensor. Under the condition that a plurality of (e.g., 3) first windows 53 are provided in the side surface of the housing, a plurality of bases 6 or only one base 6 may be provided at the position facing the first windows 53. The plurality of first windows 53 may be located on the same side or on different sides of the housing. The number of the first window 53 and the positional relationship among the windows are not limited in the present disclosure. Similarly, the base 6 can be arranged at a position according to the position of the first window 53.

In a possible embodiment, the base 6 may be provided with no hole (e.g., as shown in Figs. 15a to 15d). Alternatively, a though hole may be provided in the base 6 (e.g., as shown in Fig. 16), the through hole being in communication with the hollow cavity 4. The through hole is passed therethrough a wire/optical fiber connected with the sensor 112 for transferring a signal detected by the sensor 112, etc., which facilitates the wiring. The through hole also enables balanced pressure inside and outside the hollow cavity 4, thereby improving the accuracy of the detection of the parameters such as the pressure intensity. The position of the through hole may correspond to the position of the sensor, for example, below the sensor.

Fig. 16 is a structural chart of the monitoring probe 11 according to an embodiment of the present disclosure. As shown in Fig. 16, the base 6 may be of an L-shaped structure. The L-shaped structure may include a vertical portion 61 and a horizontal portion 62 connected with each other. The horizontal portion is disposed below the first window 53. The sensor 112 is provided on the horizontal portion. The L-shaped structure enables the overall weight of the monitoring probe 11 and the compression to the surrounding tissue to further reduce, as well as saves the materials, and so on. In addition, when the horizontal portion 62 is provided with the above-mentioned through hole, the L-shaped structure facilitates the penetration of the wire/optical fiber through the through hole and realizes the communication between the through hole and the hollow cavity 4.

In a possible embodiment, the horizontal portion 62 of the base 6 may be provided with no hole, alternatively, as shown in Fig. 16, the horizontal portion 62 of the base 6 is provided with the through hole in communication with the hollow cavity 4. For example, the through hole may be located below the sensor 112.

In a possible embodiment, a wire/optical fiber passes through the through hole arranged in the base 6 shown in Fig. 16. The wire/optical fiber is connected to the sensor 112, for transferring the signal detected by the sensor 112. The through hole further allows balanced pressure inside and outside the hollow cavity, so as to improve the accuracy of the detection of the parameters such as the pressure intensity.

The window may be provided at different positions of the housing 111 according to various detection needs. The position of the base 6 is flexibly arranged according to the position of the window, so as to realize better support and protection for the sensor 112, thereby improving the detection effect.

Figs. 17 to 20 are structural charts of the monitoring probe 11 according to an embodiment of the present application. As shown in Figs. 17 to 20, a second window 14 may be provided on the leading end of the housing 111. As indicated by the dotted lines in Figs. 17 to 20, a packaging layer may be provided at the second window 14. The packaging layer may have a streamline profile to facilitate entrance of the monitoring probe 11 into the tissue.

In a possible embodiment, as shown in Fig. 17, the sensor 112 may be fitted at the second window 14. In this case, the base may be omitted.

In a further possible embodiment, a base 6 may be provided at the second window 14, the sensor 112 being provided on the base 6, as shown in Figs. 18 to 20.

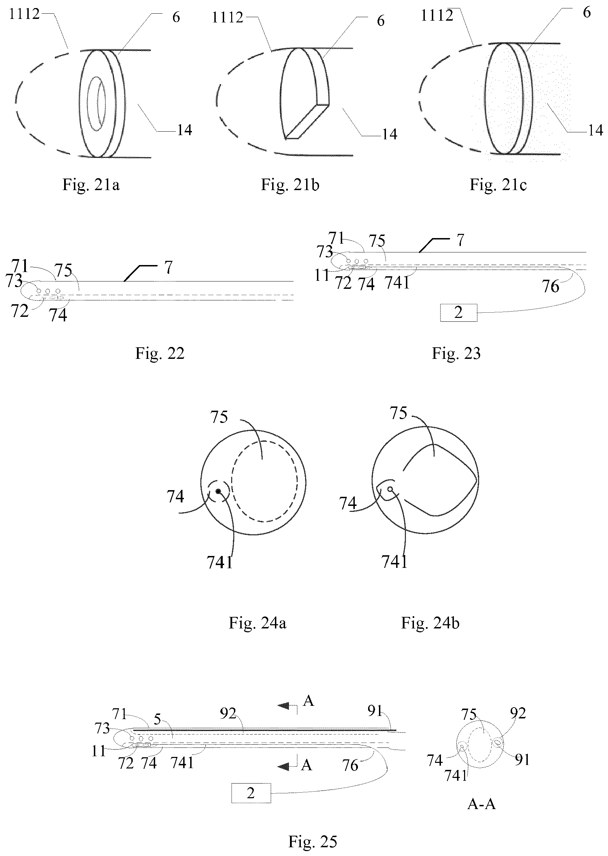

Figs. 21a to 21c are structural charts of the base on the leading end of the monitoring probe 11 according to an embodiment of the present disclosure. As shown in Figs. 21a to 21c, the base may be a plate structure matching the second window 14. That is, as shown in Figs. 18 and 21c, the shape and the dimension of the base 6 match with those of the second window 14, so as to completely cover the second window 14. The base 6 may be further provided with a through hole, as shown in Figs 19 and 21a, the position and the number of the through hole not limited. The base 6 may partially cover the second window 14, such as the structure shown by Figs. 20 and 21b. For example, when the second window 14 is circular, the base may have a defective circular shape, such as semi-circular or fan-shaped. The missing part of the circular shape serves for the same function with the through hole. The through hole and the uncovered part of the second window 14 may be passed through by a wire/optical fiber connected to the sensor 2 for transferring the signal detected by the sensor. The through hole and the uncovered part of the second window 14 also allow balanced pressure inside and outside the hollow cavity.

By providing the window on the leading end of the housing 111, the detection range is expanded. For example, the sensor may be provided near the second window 14; the packaging layer covering the second window 14 is designed to have a substantially hemispherical shape (as shown in Figs. 17 to 20), so as to expand the detection angle to 180 degrees or even more, thereby improving the detection effect.

In a possible embodiment, the monitoring bougie 1 comprises a second wireless communication module located close to an end of the monitoring bougie, which is connected to the monitoring probe 11 and is configured to transmit the collected signal in a wireless manner.

The second wireless communication module may be one or more of a Bluetooth communication module, a WiFi or IR transmission module and communicates with a device in a wireless manner. The device may include a module corresponding to the wireless communication module for receiving data transmitted by the wireless communication module. Within a certain spatial range, the wireless communication module is capable of signal exchange with the device accurately and continuously.

In a possible embodiment, the monitoring bougie 1 further comprises a processing module connected to the monitoring probe 11 and the second wireless communication module and configured to process the collected signal acquired by the monitoring probe 11 and provide the collected signal to the second wireless communication module. The second wireless communication module may transmit a sampled signal obtained by the processing.

The processing module may perform any processing for the sampled signal as appropriate. Such a processing is realizable based on the existing technical mans. For example, the processing module 15 may perform a series of post processing on the collected signal, including weak signal filtration and noise reduction, primary amplification, storage, A/D conversion, post processing and amplification, comparison with the preset threshold value, and so on. Therefore, the processing module 15 may include a filter, an amplifier, an A/D converter, a memory, a comparator, and corresponding feedback circuit, etc.

By providing at one end of the monitoring bougie the second wireless communication module, the monitoring bougie according to the embodiment of the present disclosure can connect to a monitor or a monitoring apparatus without a wire after being implanted into the body. That is, it can wirelessly transmit the collected signal to the monitor or other apparatus, so that the monitored person and the medical personnel are not subjected to the limitations of the motion range. The monitored person can move freely, without affecting the monitoring.

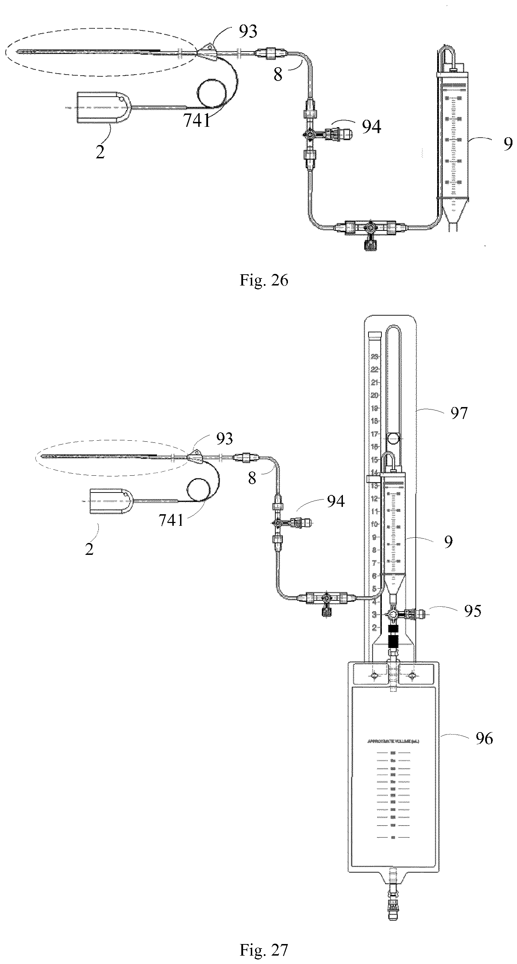

The present disclosure further provides a drainage device. Fig. 27 is a schematic diagram of the drainage device according to an embodiment of the present disclosure. As shown in Fig. 27, the drainage device comprises a drainage tube 7 (the part framed by the dotted lines in Fig. 27), the afore-described monitoring bougie 11 (not shown), a catheter 8 and a drainage bottle 9.

Fig. 22 is a schematic diagram of the drainage tube according to an embodiment of the present disclosure. As shown in Fig. 22, the drainage tube 7 mainly includes a tube body 71, the tube body 71 having a side face provided with a first through hole 72 and a second through hole 73.

The tube body 71 is provided therein more than two cavities, at least one of the cavities being a wire cavity 74 and at least one of the cavities being a drainage cavity 75.

The first through hole 72 is in communication with the wire cavity 74. The second through hole 73 is in communication with the drainage cavity 75.

The monitoring probe 11 (not shown) is provided in the wire cavity 74 (not shown) of the drainage tube 7 and connects with the monitoring apparatus 2 by means of a second wire 741 (see Fig. 23) provided in the wire cavity 74.

The catheter 8 connects the drainage tube 7 with the drainage bottle 9.

In a possible embodiment, the tube body 71 of the drainage tube may be produced by a material of biological compatibility, such as silica gel, etc. In order to prevent retrograde infection of cerebrospinal fluid, the material of the tube body 71 may be selected as antibacterial materials. The leading end portion of the tube body 71 may have a smooth streamline profile, with a leading end being a closed structure, and does not have apparent sharp edges and corners, so as to avoid injuring the surrounding tissue during the implantation or removal. For example, the tube body 71 may have a round blunt head, so as to smoothly enter the interior of a tissue such as blood vessel. Alternatively, the tube body 71 may have any shape as needed, for example, a flat structure and the like.

Fig. 23 is a schematic diagram of the drainage tube used for drainage according to an embodiment of the present disclosure. As shown in Fig. 23, in a possible embodiment, the first through hole 72 and/or the second through hole 73 may be located on a side face of the leading end portion of the tube body 71. The monitoring probe 11 is provided in the leading end portion. For example, the monitoring probe 11 may be provided in the wire cavity 74. The first through hole 72 is in communication with the wire cavity 74. The sensor on the monitoring probe 11 directly faces the first through hole 72 and monitors the physiological parameters through the first through hole 72. The leading end portion may have a sufficient length such that the monitoring probe 11 is completely inserted into the tissue.

The monitoring probe 11 is connected to the monitoring apparatus 2 in the exterior of the drainage tube by means of the second wire 741 in the wire cavity 74. In a possible embodiment, the drainage tube body 71 is provided with a third through hole 76 in communication with the wire cavity 74, the third through hole 76 drawing the second wire 741. The third through hole 76 may be arranged at a distal end of the drainage tube. For example, the second wire 741 extends along the wire cavity 74 from the leading end portion of the drainage tube to the distal end of the drainage tube, and is drawn out of the third through hole 76 to be connected to the monitoring apparatus 2 in the exterior of the drainage tube. The drainage tube may have a length from 20cm to 100cm.

The first through hole 72 may be one or more. For example, a large through hole may be provided so that all the windows of the monitoring probe 11 are capable of communicating with the exterior of the drainage tube, so as to monitor the physiological parameters. There may be provided a plurality of first through holes 72 respectively corresponding to the window(s) of the monitoring probe. The present disclosure is not limited hereto.

The second through hole 73 may serve as a drainage hole and is in communication with the drainage cavity 75. For example, as shown in Fig. 23, the leading end portion of the drainage tube may include in a vicinity of the drainage cavity 75 a drainage hole area where one or more drainage holes are provided. For example, 2-12 second through holes 73 may be distributed in the drainage hole area. The arrangement and the number of the second through hole 73 may be adjusted according to the shape and the dimension of the drainage tube and /or the drainage cavity, as long as liquid can be timely drained. The drainage hole area may be provided with developing materials that are evenly distributed over the entire drainage hole area or extends along a radial direction of the drainage tube in a band shape. The developing materials are used to determine whether the insertion position of the drainage tube is correct. For example, a developing detector is used to check whether the drainage tube is inserted in a predetermined position.

In a possible embodiment, the first through hole 72 and the second through hole 73 may each have a circular shape, an elliptical shape, a square shape, etc. The shape of the first through hole 72 and the second through hole 73 is not limited in the present disclosure. For example, the shape may be an irregular shape.

In a possible embodiment, the wire cavity 74 and the drainage cavity 75 communicate or do not communicate with each other. Fig. 24a and Fig. 24b are sectional views of the drainage tube according to an embodiment of the present disclosure. As shown in Fig. 24a, the wire cavity 74 is provided on an inner side close to a wall of the drainage tube. The wire cavity 74 and the drainage cavity 75 may be completely separated and in no communication. The second wire may be embedded in the wire cavity 74 or is movable relative to the wire cavity 74. Alternatively, as shown in Fig. 24b, the wire cavity 74 and the drainage cavity 75 may be in communication. In this case, the wire cavity 74 may be non-closed. For example, it may be a half or three fourth of a circle. The second wire 741 is engaged in the wire cavity 74 by fitting so as not to cause the drainage cavity 75 to be blocked during the drainage. Furthermore, under the condition that effective drainage is guaranteed, a plurality of the wire cavity 74 may be provided. The configuration of the wire cavity 74 and the drainage cavity 75 according to the present disclosure, it is possible to avoid blockage of the drainage cavity caused by the monitoring means or the wire connecting the monitoring means which affects the drainage effect, and is also possible to ensure that the monitoring means sufficiently contacts the environment in the tissue and accurately monitors the parameters in the tissue.

In a possible embodiment, the wire cavity 74 and drainage cavity 75 may each have a sectional shape of a circular shape, an elliptical shape, etc., or an irregular shape, which is not limited in the present disclosure. With an example where the drainage tube, the wire cavity 74 and the drainage cavity 75 each have a circular sectional shape, the drainage tube may have an outer diameter of 2.8 mm to 5.0 mm; the wire cavity 74 may have an inner diameter of 0.4 mm to 1.5 mm; and the drainage cavity 75 may have an inner diameter of 1.0 mm to 3.0 mm.

In a possible embodiment, in order to facilitate ventricular puncture, a support means 91 may be provided in the drainage tube. Fig. 25 is a schematic diagram of the drainage tube according to an embodiment of the present disclosure. As shown in Fig. 25, the support means 91 may be provided in a cavity 92 in no communication with the drainage cavity 75 and the wire cavity 74, so as to avoid infection of the tissue. The support means 91 may be a metallic support means. A leading end of the support means 91 is capable of reaching the leading end portion of the drainage tube. A distal end of the support means 91 may extend outside the drainage tube through a reserved hole in the wall of the drainage tube. For example, the support means 91 may extend out of the drainage tube through a hole in a distal end wall of the tube body 71. Hence, the support means 91 is removable when the puncture reach the preset position, so as to avoid damage to the surrounding tissue due to the support means 91. Moreover, the cavity 92 is in no communication with the other cavities, which prevents infection.

In a possible embodiment, the leading end portion of the tube body is provided with graduations. The medical personnel may intuitively determine the insertion position according to the graduations, so that the insertion position becomes more accurate, which facilitates the collection of the monitoring data and the adjustment of the drainage position.

By providing the special wire cavity 74 for drawing the second wire 741 connected to the monitoring probe 11 out of the drainage tube, and connecting the second wire 741 with the monitoring apparatus 2, the monitoring of the physiological parameters of a living body is realized. Moreover, by means of the drainage cavity 75 provided in the drainage tube, hematocele/hydrops in the body is drained to the drainage bottle 9. According to the embodiment of the present disclosure, it is possible to avoid blockage of the drainage cavity due to the wire, thereby improve the drainage effect and the accuracy of the monitoring data.

Fig. 26 is a schematic diagram of the drainage device according to an embodiment of the present disclosure. In a possible embodiment, the drainage tube is provided with a Y-shaped member 93. As shown in Fig. 26, for example, the Y-shaped member 93 may be provided at the distal end of the drainage tube. In the Y-shaped member 93, the second wire 741 is drawn out of the third through hole 76 in the wall of the drainage tube. The second wire 741, after being drawn out, is connected to the monitoring apparatus 2. The Y-shaped member, after extending out of the drainage tube, is connected to the catheter 8 by means of a joint. The joint may be a Luer taper and the like. The Y-shaped member 93 serves to secure the second wire 741 and the drainage tube body 71 and prevent the second wire 741 from being removed from the drainage tube due to uncontrollable factors of the monitored object and other factors which affects the monitoring effect. Moreover, repeated implantation is avoided as much as possible.

In a possible embodiment, the catheter 8 is provided with a first triple valve 94 for controlling (or closing) the drainage. A plurality of first triple valves 94 may be provided in case one of them breaks down and cannot control the drainage.

In a possible embodiment, the catheter 8 enters a upper end of the drainage bottle 9, so as to avoid suckback. A venting portion is provided on the upper end of the drainage bottle 9. For example, a hydrophobic filtering membrane is provided on the upper end of the drainage bottle 9 for gas to pass through, so that the drainage bottle 9 is in communication with the atmosphere during the drainage process, thereby avoiding suckback. The drainage bottle 9 may use a PVC (Polyvinyl chloride) material and the like. PVC is a transparent or translucent material. Moreover, graduations are provided on the drainage bottle 9. Hence, the drainage rate can be observed through the drainage bottle 9.

In a possible embodiment, a second triple valve 95 is provided at a lower end of the drainage bottle 9. Fig. 27 is a schematic diagram of the drainage device according to an embodiment of the present disclosure. As shown in Fig. 27, when the second triple valve 95 is closed, the medical personnel may read the graduations of the drainage bottle 9 to obtain the volume of the hematocele/hydrops drained within a certain period from the body of the patient and observe the characteristics of the hematocele/hydrops of the patient, so as to evaluate the condition of the patient. Alternatively, in some circumstances where a relatively high or relatively low pressure value inside the tissue is acquired by the monitoring apparatus 2, the drainage speed of the hematocele/hydrops in the drainage bottle 9 can be observed and adjusted by the second triple valve or by adjusting a height of the drainage bottle 9 on the supporting holder 97, so as to maintain the pressure value in the tissue within a normal value range. Meanwhile, when the patient has an excessive amount of hematocele/hydrops, in order to avoid sudden rapid drainage or excessive drainage, the hematocele/hydrops in the drainage bottle 9 may be discharged by means of cooperation between the two triple valves on the pipeline. For example, in order to avoid excessive drainage, the first triple valve 94 may be closed while the second triple valve 95 is opened to discharge the hematocele/hydrops.

In a possible embodiment, as shown in Fig. 27, the drainage bottle 9 is connected to the drainage bag 96 by way of the second triple valve 95, so as to drain the hematocele/hydrops discharged by the drainage bottle 9 into the drainage bag 96. The drainage bag 96 is provided to be easily removable and is re-attached for use after the collected hematocele/hydrops is cleaned. Alternatively, a joint may be mounted to an end of the drainage bag 96, the hematocele/hydrops being discharged at the joint.

In a possible embodiment, the drainage bottle 9 is secured on the supporting holder 97. The supporting holder 97 is provided with graduations. The medical personnel is enabled to control the flow rate of the drainage or control the pressure value by adjusting the height of the drainage bottle 9 on the supporting holder 97 according to the observed drainage and physiological monitoring conditions.

Although the embodiments of the present disclosure have been described above, it will be appreciated that the above descriptions are merely exemplary, but not exhaustive; and that the disclosed embodiments are not limiting. A number of variations and modifications may occur to one skilled in the art without departing from the scopes and spirits of the described embodiments. The terms in the present disclosure are selected to provide the best explanation on the principles and practical applications of the embodiments and the technical improvements to the arts on market, or to make the embodiments described herein understandable to one skilled in the art.

## Claims

1. A monitoring apparatus comprising:
a first interface capable of detachably connecting to a monitoring bougie;
a signal processing module configured to receive and process a collected signal transmitted by the monitoring bougie to obtain a processed signal; and
a display module configured to display the processed signal.

2. The monitoring apparatus according to claim 1, further comprising a control panel configured to enable an operator to set one or more of zero setting data, patient information, an operation mode, a warning threshold value, and warning cancellation/delay.

3. The monitoring apparatus according to claim 1, further comprising one or both of:
a wired communication module capable of communication with a first terminal in a wired manner, and
a first wireless communication module capable of communication with the first terminal in a wireless manner.

4. The monitoring apparatus according to claim 2, wherein the control panel comprises an operation mode selector switch configured to control a communication method between the monitoring apparatus and a first terminal.

5. The monitoring apparatus according to claim 1, further comprising a first storage module configured to store the processed signal when a communication with a first terminal is interrupted.

6. The monitoring apparatus according to claim 1, further comprising a first warning module configured to generate a warning signal to issue a warning according to a relationship between the processed signal and a warning threshold value.

7. The monitoring apparatus according to claim 6, wherein the first warning module is configured to issue no warning when a communication connection is established between the monitoring apparatus and a first terminal, and
the first terminal comprises a second warning module configured to, when the communication connection is established between the monitoring apparatus and the first terminal, generate a warning signal to issue a warning according to the relationship between the processed signal and the warning threshold value, or issue a warning according to the warning signal provided by the monitoring apparatus.

8. The monitoring apparatus according to claim 1, further comprising a power module configured to supply power for the monitoring apparatus and the monitoring bougie.

9. The monitoring apparatus according to claim 1, wherein the monitoring apparatus has a dimension of a length ≤ 7 cm, a width ≤ 5 cm, and a thickness ≤ 2 cm.

10. The monitoring apparatus according to claim 1, wherein the display module is further configured to display one or more of data on the collected signal, a waveform of the processed signal, error information, a power level, warning information, service time of the monitoring bougie, an ID of the monitoring bougie, patient information, and information on a wired/wireless connection.

11. The monitoring apparatus according to claim 3, wherein the monitoring apparatus and the first terminal are configured to establish a communication connection in a broadcast or handshake manner.

12. The monitoring apparatus according to claim 2, wherein the monitoring apparatus further comprises a voice input module, the control panel further comprises a voice button, and the voice button and the voice input module are configured to record a voice.

13. The monitoring apparatus according to claim 1, wherein the monitoring apparatus is wearable and is configured to fix to a head or a body of a monitored object by means of an attachment accessory.

14. The monitoring apparatus according to claim 3, further comprising:
a receiving module configured to receive the collected signal transmitted by the monitoring bougie; and
a gating module connected to the receiving module, the wired communication module and the first wireless communication module and configured to transfer the collected signal received by the receiving module to one of the wired communication module and the first wireless communication module, so as to transmit the collected signal to the first terminal.

15. The monitoring apparatus according to claim 14, wherein the gating module comprises:
a first detection module configured to detect whether a wired communication connection between the wired communication module and the first terminal is normal; and
a first selection module connected to the first detection module and configured to transfer the collected signal to the wired communication module if the first detection module detects that the wired communication connection between the wired communication module and the first terminal is normal.

16. The monitoring apparatus according to claim 15, wherein the gating module further comprises:
an enable/disable module connected to the first detection module and configured to enable the first wireless communication module and transmit a command to a second detection module if the first detection module detects that the wired communication connection between the wired communication module and the first terminal is abnormal; and
the second detection module connected to the enable/disable module and configured to detect whether the wireless communication connection between the first wireless communication module and the first terminal is normal in response to the command from the enable/disable module.

17. The monitoring apparatus according to claim 16, wherein the gating module further comprises:
a second selection module connected to the second detection module and configured to transfer the collected signal to the first wireless communication module if the second detection module detects that the wireless communication connection between the first wireless communication module and the first terminal is normal.

18. The monitoring apparatus according to claim 16, wherein the enable/disable module is connected to the second detection module and is configured to disable the first wireless communication module if the second detection module detects that the wireless communication connection between the first wireless communication module and the first terminal is abnormal.

19. The monitoring apparatus according to claim 16, further comprising an adjustment module connected to the first detection module, the second detection module and the receiving module,
and configured to:
cause the receiving module to sample the collected signal at a first sampling frequency if the first detection module detects that the wired communication connection between the wired communication module and the first terminal is normal, and
cause the receiving module to sample the collected signal at a second sampling frequency if the first detection module detects that the wired communication connection between the wired communication module and the first terminal is abnormal and the second detection module detects that the wireless communication connection between the first wireless communication module and the first terminal is normal,
wherein the first sampling frequency is higher than the second sampling frequency.

20. The monitoring apparatus according to claim 18, wherein the enable/disable module is configured to disable the first wireless communication module and regularly enable the first wireless communication module if the second detection module detects that the wireless communication connection between the first wireless communication module and the first terminal is abnormal.

21. The monitoring apparatus according to any one of claims 14 to 19, wherein the first wireless communication module comprises a Bluetooth communication module.

22. A monitoring bougie comprising:
a monitoring probe configured to acquire a collected signal;
a connector configured to have one end connected to the monitoring probe via a first wire and one other end having a second interface detachably connectable to the first interface of the monitoring apparatus according to any one of claims 1 to 21; and
a second storage module configured to store an ID of the monitoring bougie and a preset threshold value.

23. The monitoring bougie according to claim 22, wherein the monitoring probe comprises a sensor for collecting the collected signal.

24. The monitoring bougie according to claim 22, wherein the second storage module is disposed in the monitoring probe, or in the connector, or between the monitoring probe and connector.

25. The monitoring bougie according to claim 22, wherein the monitoring probe has a diameter ≤800 µm.

26. The monitoring bougie according to claim 23, wherein the sensor is a piezoelectric or piezoresistive sensor with a circuit of a full-bridge connection or a half-bridge connection, and configured to measure an absolute pressure or a relative pressure.

27. The monitoring bougie according to claim 22, wherein the second storage module is a memory chip unaffected by magnetic resonance (MR) and computed tomography (CT).

28. The monitoring bougie according to claim 22, wherein the second storage module is further configured to store one or more of zero setting data, patient information, time of implantation, a factory version of the monitoring bougie, and the collected signal.

29. The monitoring bougie according to claim 22, further comprising an electronic identity tag.

30. The monitoring bougie according to claim 23, wherein the monitoring probe further comprises a housing, a window being opened on a surface of the housing, and the sensor being disposed at a position facing the window.

31. The monitoring bougie according to claim 30, wherein a packaging layer is provided at the window.

32. The monitoring bougie according to claim 30 or 31, wherein one or more windows are opened on the surface of the housing.

33. The monitoring bougie according to claim 32, wherein one or more sensors are provided at a position facing the same window.

34. The monitoring bougie according to claim 32, wherein the sensor is provided at each of positions facing a plurality of windows.

35. The monitoring bougie according to claim 33 or 34, wherein the plurality of sensors are separated from one another.

36. The monitoring bougie according to claim 33 or 34, wherein the plurality of the sensors are integrated as one component.

37. The monitoring bougie according to claim 32, wherein the one or more windows are disposed on one or more side surfaces of the housing.

38. The monitoring bougie according to claim 37, wherein the windows are disposed on opposite side surfaces of the housing.

39. The monitoring bougie according to claim 38, wherein the windows are disposed on opposite side surfaces of the housing in a staggered arrangement.

40. The monitoring bougie according to claim 30, wherein the window is disposed on a leading end of the housing.

41. The monitoring bougie according to claim 40, wherein a packaging layer provided at the window disposed on the leading end has a streamline profile.

42. The monitoring bougie according to claim 30, wherein a hollow cavity in communication with the window is provided in the housing.

43. The monitoring bougie according to claim 42, further comprising a base provided in the hollow cavity, the sensor being provided on the base.

44. The monitoring bougie according to claim 43, wherein a first window is opened in a side surface of the housing, and the base is provided in the hollow cavity on a side facing the first window.

45. The monitoring bougie according to claim 44, wherein the base is of an L-shaped structure, the L-shaped structure including a vertical portion and a horizontal portion connected with each other, the horizontal portion being disposed below the first window, the sensor being provided on the horizontal portion.

46. The monitoring bougie according to claim 42, wherein a second window is opened on a leading end of the housing.

47. The monitoring bougie according to claim 46, further comprising a base provided at the second window, the sensor being provided on the base.

48. The monitoring bougie according to claim 47, wherein the base is of a plate-like structure matching the second window.

49. The monitoring bougie according to any one of claims 43 to 45, 47 and 48, wherein the base is provided with a through hole in communication with the hollow cavity.

50. The monitoring bougie according to claim 49, wherein the through hole is penetrated by a wire/optical fiber, the wire/optical fiber being connected with the sensor.

51. The monitoring bougie according to claim 47, wherein the base partially covers the second window.

52. The monitoring bougie according to any one of claims 43 to 45, 47 and 48, wherein the base is provided with no hole.

53. The monitoring bougie according to claim 46, wherein the sensor is fitted at the second window.

54. The monitoring bougie according to claim 22, further comprising a second wireless communication module disposed close to an end of the monitoring bougie, connected to the monitoring probe, and configured to wirelessly transmit the collected signal.

55. The monitoring bougie according to claim 54, further comprising a processing module connected to the monitoring probe and the second wireless communication module, and configured to process the collected signal acquired by the monitoring probe and provide the processed collected signal to the second wireless communication module.

56. A monitoring system comprising:
a first terminal;
the monitoring apparatus according to any one of claims 1 to 21; and
the monitoring bougie according to any one of claims 22 to 55,
wherein the monitoring apparatus is configured to receive and process the collected signal transmitted by the monitoring bougie to obtain the processed signal, and transmit the processed signal to the first terminal, and
the first terminal is configured to receive the processed signal from the monitoring apparatus, analyze the processed signal to acquire monitoring data, and display the monitoring data.

57. The monitoring system according to claim 56, further comprising a server connecting to the first terminal and receiving the monitoring data transmitted by the first terminal.

58. The monitoring system according to claim 57, wherein the server is configured to connect to a second terminal, and the second terminal is capable of acquiring the monitoring data through the server.
